(19) Europäisches Patentamt / European Patent Office / Office européen des brevets

(11) EP 4 328 046 A1

(12) EUROPEAN PATENT APPLICATION
published in accordance with Art. 153(4) EPC

(43) Date of publication:
**28.02.2024 Bulletin 2024/09**

(21) Application number: **22804240.4**

(22) Date of filing: **02.02.2022**

(51) International Patent Classification (IPC):
***B60C 1/00*** *(2006.01)* ***C07C 291/06*** *(2006.01)*
***C08C 19/22*** *(2006.01)* ***C08F 36/04*** *(2006.01)*
***C08L 15/00*** *(2006.01)*

(52) Cooperative Patent Classification (CPC):
**B60C 1/00; C07C 291/06; C08C 19/22; C08F 36/04; C08L 15/00;** Y02T 10/86

(86) International application number:
**PCT/JP2022/003943**

(87) International publication number:
**WO 2022/244317 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.05.2021 JP 2021083339**

(71) Applicant: **Sumitomo Rubber Industries, Ltd.**
**Kobe-shi, Hyogo 651-0072 (JP)**

(72) Inventor: **JIANG, Lan**
**Kobe-shi, Hyogo 651-0072 (JP)**

(74) Representative: **Manitz Finsterwald**
**Patent- und Rechtsanwaltspartnerschaft mbB**
**Martin-Greif-Strasse 1**
**80336 München (DE)**

# (54) NITRILE OXIDE COMPOUND AND IONIC-FUNCTIONAL-GROUP-CONTAINING POLYMER

(57) Provided are a novel nitrile oxide compound containing a nitrile oxide group and an ionic functional group that can react with various unsaturated polymers, an ionic functional group-containing polymer into which the nitrile oxide compound has been introduced, and a rubber composition and a tire each produced using the ionic functional group-containing polymer.

FIG.1

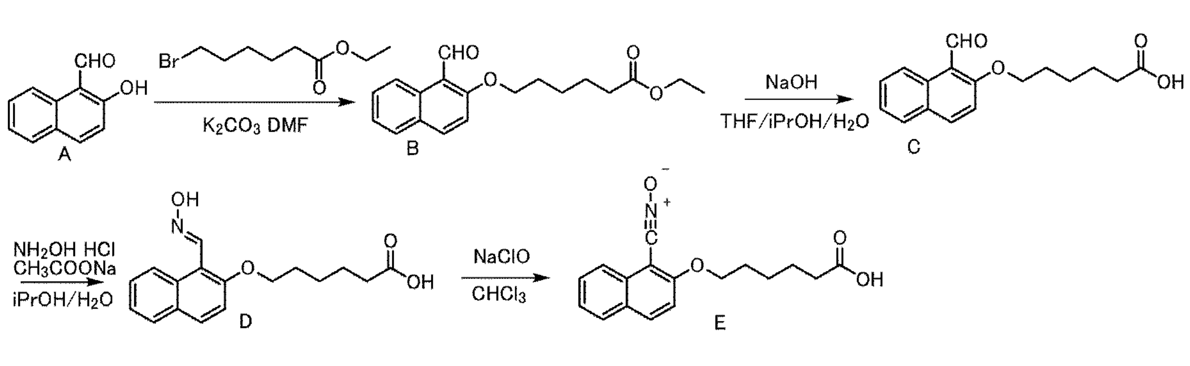


EP 4 328 046 A1

## Description

### TECHNICAL FIELD

The present invention relates to a nitrile oxide compound, an ionic functional group-containing polymer, a rubber composition, and a tire.

### BACKGROUND ART

Ionic molecule-containing rubbers have been disclosed as polymers having various properties, and introduction of a carboxylic acid-containing unit into a styrene-butadiene rubber has been proposed, for example. However, it is commonly difficult to adjust the amount of an ionic functional group such as a carboxylic acid group in a polymer or to introduce ionic functional groups into various polymers.

### SUMMARY OF INVENTION

### TECHNICAL PROBLEM

In response to the demand for a technique for easily introducing a low-molecular-weight compound containing an ionic functional group such as a carboxylic acid group into a polymer, the present inventors focused on a nitrile oxide compound and synthesized a novel nitrile oxide compound containing a nitrile oxide group and an ionic functional group. The present inventors found out that this novel compound can react with various unsaturated polymers, does not especially require a catalyst in synthesis thereof, and can suppress the production of by-products. Thus, the present invention was completed.

The present invention aims to solve the above problems and to provide a novel nitrile oxide compound containing a nitrile oxide group and an ionic functional group that can react with various unsaturated polymers, an ionic functional group-containing compound into which the nitrile oxide compound has been introduced, and a rubber composition and a tire each produced using the ionic functional group-containing polymer.

### SOLUTION TO PROBLEM

The present invention relates to a nitrile oxide compound containing a nitrile oxide group and an ionic functional group.

### EFFECTS OF INVENTION

Since a nitrile oxide compound of the present invention contains a nitrile oxide group and an ionic functional group, the present invention can provide a novel nitrile oxide compound containing a nitrile oxide group and an ionic functional group that can react with various unsaturated polymers, an ionic functional group-containing polymer into which the nitrile oxide compound has been introduced, and a rubber composition and a tire each produced using the ionic functional group-containing polymer.

### BRIEF DESCRIPTION OF THE DRAWINGS

FIG. 1 shows a diagram illustrating an example of a method for synthesizing a nitrile oxide compound containing a nitrile oxide group and an ionic functional group.

FIG. 2 shows an example of $^1$H-NMR (proton NMR) spectra of compounds C, D, and E in the total route in FIG. 1.

FIG. 3 shows an example of IR spectra of the compounds D and E in the total route in FIG. 1.

FIG. 4 shows an example of synthesis in which a nitrile oxide compound containing a nitrile oxide group and an ionic functional group reacts with a polymer containing a double bond into a polymer containing an ionic functional group.

FIG. 5 shows an example of $^1$H-NMR (proton NMR) spectra of polymers in the total route in FIG. 4.

FIG. 6 shows a diagram illustrating an example of a method for synthesizing a nitrile oxide compound containing a nitrile oxide group and an ionic functional group.

FIG. 7 shows a diagram illustrating an example of a method for synthesizing a nitrile oxide compound containing a nitrile oxide group and an ionic functional group.

FIG. 8 shows an example of IR spectra of Example 1-1 in which SSBR1-4 (ionic functional group-containing SBR) is used and Comparative Example 1-1 in which corresponding SSBR1 is used and a difference spectrum of them.

DESCRIPTION OF EMBODIMENTS

<Nitrile oxide compound>

The present invention relates to a novel nitrile oxide compound containing a nitrile oxide group and an ionic functional group. Since the nitrile oxide compound can react with various polymers, it enables simple introduction of the ionic functional group into a polymer, thereby providing an ionic functional group-containing polymer. In addition, the nitrile oxide compound does not especially require a catalyst in synthesis thereof, which suppresses the production of by-products.

The nitrile oxide group in the nitrile oxide compound containing a nitrile oxide group and an ionic functional group is represented by the following formula. The nitrile oxide compound may contain one nitrile oxide group or two or more nitrile oxide groups.

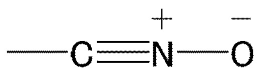

In the nitrile oxide compound containing a nitrile oxide group and an ionic functional group, the ionic functional group may be, for example, a cationic functional group or an anionic functional group. Desirably, the ionic functional group has been introduced to a terminal of the nitrile oxide compound. The nitrile oxide compound may contain one ionic functional group or two or more ionic functional groups. Also, the nitrile oxide compound may contain one type of ionic functional group(s) or two or more types of ionic functional groups.

Examples of the cationic functional group include basic functional groups. Examples of the basic functional groups include an amino group, an imino group (=NH), an ammonium base, and a heterocyclic group containing a basic nitrogen atom. The amino group may be a primary amino group ($-NH_2$), a secondary amino group ($-NHR^1$), or a tertiary amino group ($-NR^1R^2$). $R^1$ and $R^2$ each may be, for example, an alkyl group, a phenyl group, or an aralkyl group. The number of carbon atoms of each of $R^1$ and $R^2$ is preferably 1 to 8. The ammonium base may be, for example, a tertiary ammonium base or a quaternary ammonium base. The heterocyclic group containing a basic nitrogen atom may be, for example, a nitrogen-containing heterocyclic group such as a pyridine group, a pyrimidine group, a pyrazine group, an imidazole group, a thiol-containing imidazole group, a triazole group, or a thiazole group. Preferred among these is an amino group (primary amino group, secondary amino group, or tertiary amino group).

Examples of the anionic functional group include halogen groups and acidic functional groups. Examples of the halogen groups include a fluoro group, a chloro group, a bromo group, and an iodo group. Examples of the acidic functional groups include a hydroxy group, a carboxylic acid group, a sulfonic acid group, a sulfuric acid group, a phosphonic acid group, a phosphoric acid group, a phosphinic acid group, a maleic acid group, an acid anhydride group (e.g., maleic anhydride group), a fumaric acid group, an itaconic acid group, an acrylic acid group, a methacrylic acid group, and a mercapto group. Preferred among these is a carboxylic acid group (carboxy group).

The nitrile oxide compound may be any compound containing a nitrile oxide group and an ionic functional group, and may be, for example, a compound represented by the following formula:

$$X\text{-}C{\equiv}N^{+}\text{-}O^{-}$$

(wherein X is a monovalent hydrocarbon group containing an ionic functional group).

X may be any monovalent hydrocarbon group containing an ionic functional group. Examples of the ionic functional group in X include the same groups as the above-mentioned ionic functional groups. The ionic functional group in X is preferably formed at a terminal of X as in the case where X is a group represented by $-(CH_2)_5-A$ (A: ionic functional group), for example.

The monovalent hydrocarbon group in X (a monovalent hydrocarbon group containing an ionic functional group) means a monovalent hydrocarbon group that constitutes the skeleton of X and has an ionic functional group replaced with a hydrogen atom. For example, when X is a group represented by $-(CH_2)_5-COOH$ (COOH: ionic functional group), the monovalent hydrocarbon group in X is $-(CH_2)_5-H$.

The monovalent hydrocarbon group in X may be, for example, a substituted or unsubstituted monovalent hydrocarbon group, which may be linear, branched, or cyclic and is preferably cyclic. The monovalent hydrocarbon group in X may contain a heteroatom. The substituent is not limited and may be a known group such as a hydroxy group and a halogen group (-Cl, -Br, etc.). The heteroatom is not limited, and may be oxygen, nitrogen, or the like. The

monovalent hydrocarbon group may contain one or more of these substituents and one or more of these heteroatoms.

The number of carbon atoms in the monovalent hydrocarbon group in X is preferably 3 or more, more preferably 6 or more, while it is preferably 30 or less, more preferably 26 or less, still more preferably 20 or less.

Examples of the monovalent hydrocarbon group in X include a substituted or unsubstituted linear alkyl group optionally containing a heteroatom, a substituted or unsubstituted branched alkyl group optionally containing a heteroatom, a substituted or unsubstituted cyclic alkyl group optionally containing a heteroatom, a substituted or unsubstituted aryl group optionally containing a heteroatom, and a substituted or unsubstituted aralkyl group optionally containing a heteroatom. The monovalent hydrocarbon group in X may also be a group obtained by binding these groups including a substituted or unsubstituted linear alkyl group optionally containing a heteroatom, a substituted or unsubstituted branched alkyl group optionally containing a heteroatom, a substituted or unsubstituted cyclic alkyl group optionally containing a heteroatom, a substituted or unsubstituted aryl group optionally containing a heteroatom, and a substituted or unsubstituted aralkyl group optionally containing a heteroatom (e.g., a group obtained by binding a substituted or unsubstituted linear alkyl group optionally containing a heteroatom and a substituted or unsubstituted aryl group optionally containing a heteroatom).

In the monovalent hydrocarbon group in X, examples of the substituted or unsubstituted linear alkyl group optionally containing a heteroatom and the substituted or unsubstituted branched alkyl group optionally containing a heteroatom include a methyl group, an ethyl group, an n-propyl group, an isopropyl group, an n-butyl group, an isobutyl group, a sec-butyl group, a tert-butyl group, a pentyl group, a hexyl group, a heptyl group, a 2-ethylhexyl group, an octyl group, a nonyl group, a decyl group, and the foregoing groups containing heteroatoms. Examples of the substituted or unsubstituted cyclic alkyl group optionally containing a heteroatom include a cyclopropyl group, a cyclobutyl group, a cyclopentyl group, a cyclohexyl group, a cycloheptyl group, a cyclooctyl group, an adamantyl group, a 1-ethylcyclopentyl group, a 1-ethylcyclohexyl group, and the foregoing groups containing heteroatoms. Examples of the substituted or unsubstituted aryl group optionally containing a heteroatom include a phenyl group, a tolyl group, a xylyl group, a biphenyl group, a naphthyl group, an anthryl group, a phenanthryl group, and the foregoing groups containing heteroatoms. Examples of the substituted or unsubstituted aralkyl group optionally containing a heteroatom include a benzyl group, a phenethyl group, and the foregoing groups containing heteroatoms. The monovalent hydrocarbon group in X may also be a group obtained by binding these groups (e.g., a group obtained by binding a substituted or unsubstituted pentyl group optionally containing a heteroatom and a substituted or unsubstituted naphthyl group optionally containing a heteroatom).

In particular, the monovalent hydrocarbon group in X is preferably a substituted or unsubstituted linear alkyl group optionally containing a heteroatom, a substituted or unsubstituted aryl group optionally containing a heteroatom, or a group obtained by binding a substituted or unsubstituted linear alkyl group optionally containing a heteroatom and a substituted or unsubstituted aryl group optionally containing a heteroatom.

The compound containing a nitrile oxide group and an ionic functional group is preferably a compound containing a cyclic structure, more preferably a compound containing an aromatic ring. Specific examples of the aromatic ring include a benzene ring, a naphthalene ring, an anthracene ring, a phenanthrene ring, a fluorene ring, a triphenylene ring, a naphthacene ring, a biphenyl ring, a bisphenol ring, and a terphenyl ring (three benzene rings may be linked in any manner). Preferred among these is a compound containing a benzene ring or a naphthalene ring, and more preferred is a compound containing a naphthalene ring.

Suitable examples of the nitrile oxide compound containing a nitrile oxide group and an ionic functional group include a compound represented by the following formula (a compound containing a naphthalene ring):

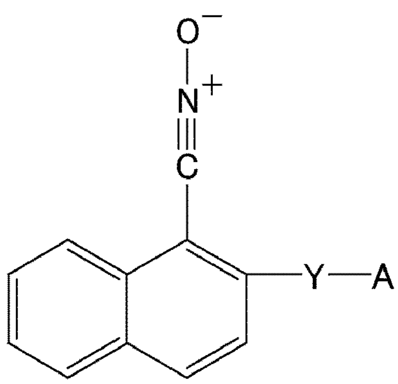

(wherein A is a monovalent ionic functional group, Y is a substituted or unsubstituted divalent hydrocarbon group optionally containing a heteroatom).

A (monovalent ionic functional group) may be, for example, any of the monovalent ionic functional groups

mentioned above.

Y (substituted or unsubstituted divalent hydrocarbon group optionally containing a heteroatom) may be linear, branched, or cyclic. The heteroatom and substituent in Y may be, for example, any of the heteroatoms mentioned above and any of the substituents mentioned above. Y may contain one or more of these substituents and one or more of these heteroatoms.

The number of carbon atoms in the divalent hydrocarbon group in Y is preferably 1 or more, more preferably 2 or more, still more preferably 3 or more, while it is preferably 20 or less, more preferably 12 or less, still more preferably 10 or less.

Y (substituted or unsubstituted divalent hydrocarbon group optionally containing a heteroatom) may be, for example, a substituted or unsubstituted alkylene group optionally containing a heteroatom, a substituted or unsubstituted alkenylene group optionally containing a heteroatom, a substituted or unsubstituted cycloalkylene group optionally containing a heteroatom, a substituted or unsubstituted cycloalkylalkylene group optionally containing a heteroatom, a substituted or unsubstituted arylene group optionally containing a heteroatom, a substituted or unsubstituted aralkylene group optionally containing a heteroatom, or a substituted or unsubstituted oxyalkylene group optionally containing a heteroatom.

Examples of the substituted or unsubstituted alkylene group optionally containing a heteroatom include a methylene group, an ethylene group, a trimethylene group, a tetramethylene group, a pentamethylene group, a hexamethylene group, an octylene group, a nonylene group, a decylene group, a 1,2-propylene group, and the foregoing groups containing heteroatoms. Examples of the substituted or unsubstituted alkenylene group optionally containing a heteroatom include a vinylene group, a 1-propenylene group, a 2-propenylene group, and the foregoing groups containing heteroatoms. Examples of the substituted or unsubstituted cycloalkylene group optionally containing a heteroatom include a cyclohexylene group and a cyclohexylene group containing a heteroatom. Examples of the substituted or unsubstituted cycloalkylalkylene group optionally containing a heteroatom include a cyclohexylmethylene group and a cyclohexylmethylene group containing a heteroatom. Examples of the substituted or unsubstituted arylene group optionally containing a heteroatom include a phenylene group, a tolylene group, a xylylene group, and the foregoing groups containing heteroatoms. Examples of the substituted or unsubstituted aralkylene group optionally containing a heteroatom include a benzylidene group and a benzylidene group containing a heteroatom. Examples of the substituted or unsubstituted oxyalkylene group optionally containing a heteroatom include an oxyethylene group, an oxypropylene group, an oxybutylene group, an oxytetramethylene group, and the foregoing groups containing heteroatoms.

The compound containing a naphthalene ring represented by the above formula is a suitable example, and any positions selected from the 1-position to the 8-position in the naphthalene ring may be substituted with the groups represented by $-C{\equiv}N^{+}-O^{-}$ and -Y-A. In particular, the 1-position and the 2-position are preferably substituted with $-C{\equiv}N^{+}-O^{-}$ and -Y-A.

Next, a method for synthesizing the nitrile oxide compound containing a nitrile oxide group and an ionic functional group will be described. An example of a method for synthesizing a nitrile oxide compound containing a nitrile oxide group and an ionic functional group will be described below. However, the nitrile oxide compound is not limited to one obtained by such a synthesis method and may be a compound obtained by any synthesis method capable of synthesizing the nitrile oxide compound.

First, a compound containing a hydroxy group and an aldehyde group, as a starting material, is reacted with a compound into which an ionic functional group can be introduced. If necessary, a group derived from the compound into which an ionic functional group can be introduced in the obtained substance is substituted with the ionic functional group.

Next, the aldehyde group in the resulting compound is converted to a group represented by -CH=N-OH, and the group represented by -CH=N-OH is further converted to a group represented by $-C{\equiv}N^{+}-O-$, whereby a nitrile oxide compound containing a nitrile oxide group and an ionic functional group can be synthesized.

The reaction process may be carried out in an organic solvent, in water, or in the absence of a solvent. The organic solvent is not limited and is preferably one in which both the compound to be reacted and the reacting compound are easily soluble. Non-limiting examples of the organic solvent include toluene, mesitylene, chloroform ($CHCl_3$), alcohol, and tetrahydrofuran (THF). If necessary, a catalyst may be used. Non-limiting examples of the catalyst include N,N-dimethylformamide (DMF), dimethyl sulfoxide (DMSO), and triethylamine (TEA). The amount of the solvent and the amount of the catalyst may be appropriately set according to the compound so that the reaction proceeds. The reaction temperature and reaction time may also be appropriately set according to the compound so that the reaction proceeds. The reaction temperature is, for example, 10°C to 100°C, preferably 20°C to 80°C and the reaction time is, for example, 1 to 200 hours, preferably 3 to 100 hours.

To illustrate a specific example of synthesizing the compound, for example, a nitrile oxide compound containing a nitrile oxide group and an ionic functional group (compound E) can be synthesized according to the synthetic route shown in FIG. 1.

First, a compound A (a compound containing a hydroxy group and an aldehyde group), as a starting material, is reacted with a compound into which an ionic functional group can be introduced ($Br-(CH_2)_5-C(=O)-O-CH_2CH_3$), whereby

a compound B is obtained. The group represented by -C(=O)-O-$CH_2CH_3$ (a group derived from the compound into which an ionic functional group can be introduced) in the resulting compound B is substituted with -C(=O)-OH (ionic functional group: carboxylic acid group), whereby a compound C is obtained.

The aldehyde group in the resulting compound C was converted to a group represented by -CH=N-OH, whereby a compound D is synthesized. The group represented by -CH=N-OH in the resulting compound D is converted to a group represented by -C≡$N^+$-O-, whereby a compound E (a nitrile oxide compound containing a nitrile oxide group and an ionic functional group) is synthesized.

FIG. 2 is an example of $^1$H-NMR (proton NMR) spectra of the compounds C, D, and E in the total route in FIG. 1. FIG. 3 is an example of IR spectra of the compounds D and E in the total route in FIG. 1. The spectra in FIG. 2 confirm the synthesis of the compounds C, D, and E and the spectra in FIG. 3 confirm the synthesis of the compounds D and E.

A nitrile oxide compound containing a nitrile oxide group and an ionic functional group shown as a compound I can be synthesized according to the synthetic route shown in FIG. 6.

First, the compound A (a compound containing a hydroxy group and an aldehyde group), as a starting material, is reacted with a compound into which an ionic functional group can be introduced (Cl-$(CH_2)_6$-OH), whereby a compound F is obtained.

The aldehyde group in the resulting compound F is converted to -CH=N-OH, whereby a compound G is synthesized. The group represented by -CH=N-OH in the resulting compound G is converted to -C≡$N^+$-O-, whereby a compound H (ionic functional group: hydroxy group) is obtained.

The group represented by -OH (a group derived from the compound into which an ionic functional group can be introduced) in the resulting compound H is substituted with -O-C(=O)-CH$(CH_3)_2$-BR (ionic functional group: halogen group), whereby a compound I (a nitrile oxide compound containing a nitrile oxide group and an ionic functional group) is synthesized.

For example, a nitrile oxide compound containing a nitrile oxide group and an ionic functional group shown as a compound e can be synthesized according to the synthetic route shown in FIG. 7.

First, a compound a (a compound containing a hydroxy group and an aldehyde group), as a starting material, is reacted with a compound into which an ionic functional group can be introduced (Br-$CH_2$-Ph-C(=O)-O-$CH_3$), whereby a compound b is obtained.

The group represented by -C(=O)-O-$CH_3$ in the resulting compound b (a group derived from the compound into which an ionic functional group can be introduced) is converted to -C(=O)-OH (ionic functional group: carboxylic acid group), whereby a compound c is obtained.

The aldehyde group in the resulting compound c is converted to -CH=N-OH, whereby a compound d is synthesized. The group represented by -CH=N-OH in the resulting compound d is converted to -C≡$N^+$-O-, whereby a compound e (a nitrile oxide compound containing a nitrile oxide group and an ionic functional group) is synthesized.

<Ionic functional group-containing polymer>

An ionic functional group-containing polymer obtained by reacting a nitrile oxide compound containing a nitrile oxide group and an ionic functional group with a polymer is a modified polymer obtainable by reacting a nitrile oxide compound containing a nitrile oxide group and an ionic functional group with a polymer (e.g., a polymer containing an unsaturated bond). Use of the ionic functional group-containing polymer improves tire performances such as wet grip performance.

The reason why the above effect can be obtained is not clear, but it may be attributed to the following mechanism.

A nitrile oxide compound containing a nitrile oxide group and an ionic functional group can react with various unsaturated polymers, and therefore is easily added to an unsaturated polymer such as a diene-based rubber, which enables easy synthesis of a polymer having an ion binding point (a site where an ionic bond is formed). Also, the ion binding point content of the polymer can be easily adjusted. For example, when a rubber composition for a tire containing a diene-based rubber into which the nitrile oxide compound has been introduced comes into contact with a wet road surface, the ion binding point is dissociated to loosen the crosslinkage, lowering the elastic modulus. Therefore, the contact area with the road surface during running on a wet road surface increases to increase the friction, which improves the loss. This is believed to improve the wet grip performance.

A method for reacting a nitrile oxide compound containing a nitrile oxide group and an ionic functional group with a polymer may be any method by which the reaction can be performed. For example, the nitrile oxide compound and a polymer containing an unsaturated bond are dissolved or energized, as needed, to cause a cycloaddition reaction between the nitrile oxide group and a carbon-carbon double bond in the backbone of the polymer, whereby a reaction to form a five-membered ring can proceed.

The polymer may be, for example, a polymer containing a carbon-carbon double bond. The polymer containing a carbon-carbon double bond may be, for example, a rubber component such as a diene-based rubber. Examples of diene-based rubbers include isoprene-based rubbers, polybutadiene rubbers (BR), styrene-butadiene rubbers (SBR),

styrene-isoprene-butadiene rubbers (SIBR), ethylene-propylene-diene rubbers (EPDM), chloroprene rubbers (CR), and acrylonitrile-butadiene rubbers (NBR). Examples also include butyl-based rubbers and fluororubbers. From the standpoint of tire performances such as wet grip performance, preferred among these are SBR, BR, and isoprene-based rubbers, and more preferred are SBR and BR.

The diene-based rubbers may include unmodified diene-based rubbers and modified diene-based rubbers.

The modified diene-based rubbers may be any diene-based rubber having a functional group interactive with a filler such as silica. Examples include a chain end-modified diene-based rubber obtained by modifying at least one chain end of a diene-based rubber by a compound (modifier) having the functional group (i.e., a chain end-modified diene-based rubber terminated with the functional group); a backbone-modified diene-based rubber having the functional group in the backbone; a backbone- and chain end-modified diene-based rubber having the functional group in both the backbone and chain end (e.g., a backbone- and chain end-modified diene-based rubber in which the backbone has the functional group and at least one chain end is modified by the modifier); and a chain end-modified diene-based rubber into which a hydroxy or epoxy group has been introduced by modification (coupling) with a polyfunctional compound having two or more epoxy groups in the molecule.

Examples of the functional group include amino, amide, silyl, alkoxysilyl, isocyanate, imino, imidazole, urea, ether, carbonyl, oxycarbonyl, mercapto, sulfide, disulfide, sulfonyl, sulfinyl, thiocarbonyl, ammonium, imide, hydrazo, azo, diazo, carboxyl, nitrile, pyridyl, alkoxy, hydroxy, oxy, and epoxy groups. These functional groups may be substituted. Preferred among these are amino groups (preferably amino groups whose hydrogen atom is replaced with a C1-C6 alkyl group), alkoxy groups (preferably C1-C6 alkoxy groups), and alkoxysilyl groups (preferably C1-C6 alkoxysilyl groups).

Non-limiting examples of the SBR include emulsion-polymerized styrene-butadiene rubber (E-SBR) and solution-polymerized styrene-butadiene rubber (S-SBR). These may be used alone or in combination of two or more.

The styrene content of the SBR is preferably 3% by mass or higher, more preferably 5% by mass or higher, still more preferably 7% by mass or higher. The styrene content is preferably 60% by mass or lower, more preferably 55% by mass or lower, still more preferably 50% by mass or lower. When the styrene content is within the range indicated above, tire performances such as wet grip performance tend to be better obtained.

Herein, the styrene content of the SBR is determined by $^1$H-NMR analysis.

The vinyl content of the SBR is preferably 3% by mass or higher, more preferably 5% by mass or higher, still more preferably 7% by mass or higher. The vinyl content is preferably 60% by mass or lower, more preferably 55% by mass or lower, still more preferably 50% by mass or lower. When the vinyl content is within the range indicated above, tire performances such as wet grip performance tend to be better obtained.

Herein, the vinyl content (1,2-bond butadiene unit content) can be determined by infrared absorption spectrometry.

SBR products manufactured or sold by Sumitomo Chemical Co., Ltd., JSR Corporation, Asahi Kasei Corporation, Zeon Corporation, etc. may be used as the SBR.

The SBR may be either unmodified or modified SBR. Examples of the modified SBR include those into which functional groups listed for the modified diene-based rubbers have been introduced. The SBR may be hydrogenated.

Any BR may be used, such as high-cis BR having a high cis content, BR containing syndiotactic polybutadiene crystals, or BR synthesized using rare earth catalysts (rare earth-catalyzed BR). These may be used alone or in combination of two or more. High-cis BR having a cis content of 90% by mass or higher is preferred to improve abrasion resistance.

The BR may be either unmodified or modified BR. Examples of the modified BR include those into which functional groups listed for the modified diene-based rubbers have been introduced. BR may be hydrogenated.

Usable commercial BR may be available from Ube Industries, Ltd., JSR Corporation, Asahi Kasei Corporation, Zeon Corporation, etc.

Examples of the isoprene-based rubbers include natural rubbers (NR), polyisoprene rubbers (IR), refined NR, modified NR, and modified IR. Examples of the NR include those commonly used in the rubber industry such as SIR20, RSS#3, and TSR20. Any IR may be used, and examples include those commonly used in the rubber industry, such as IR2200. Examples of the refined NR include deproteinized natural rubbers (DPNR) and ultra pure natural rubbers (UPNR). Examples of the modified NR include epoxidized natural rubbers (ENR), hydrogenated natural rubbers (HNR), and grafted natural rubbers. Examples of the modified IR include epoxidized polyisoprene rubbers, hydrogenated polyisoprene rubbers, and grafted polyisoprene rubbers. These may be used alone or in combination of two or more.

The reaction process between the nitrile oxide compound and the polymer such as the diene-based rubber may be carried out in any environment such as in an organic solvent, in water, or in the absence of a solvent. The organic solvent is not limited and is preferably one in which both the nitrile oxide compound and the polymer are easily soluble. Specific examples of the organic solvent include those listed above. The reaction temperature and reaction time may be appropriately set according to the nitrile oxide compound and the polymer so that the reaction proceeds.

To illustrate a specific example of synthesizing the compound, for example, a reaction product (a polymer

containing an ionic functional group) between a nitrile oxide compound containing a nitrile oxide group and an ionic functional group and a polymer containing a double bond can be synthesized according to the synthetic route shown in FIG. 4.

Specifically, a nitrile oxide compound containing a nitrile oxide group and an ionic functional group (the compound E), and BR and SBR (diene-based rubbers) are dissolved in a solvent, and the resulting solution is reacted by stirring at an appropriate temperature. After completion of the reaction, the solution containing the reaction product is subjected to precipitation in methanol, followed by drying, whereby a target reaction product (a polymer having an ionic functional group) is obtained.

FIG. 5 shows an example of $^1$H-NMR (proton NMR) spectra of polymers in the total route in FIG. 4. The spectrum (A) is an exemplary $^1$H-NMR spectrum of SBR, the spectrum (B) is an exemplary $^1$H-NMR spectrum of SBR containing COOH (ionic functional group), and the spectrum (C) is an exemplary $^1$H-NMR spectrum of BR containing COOH (ionic functional group). The spectra (B) and (C) in FIG. 5 confirm the synthesis of a polymer containing an ionic functional group by a reaction of a nitrile oxide compound containing a nitrile oxide group and an ionic functional group (the compound E) with BR and SBR.

<Rubber composition>

The rubber composition contains, as a rubber component, the ionic functional group-containing polymer obtained by reacting a nitrile oxide compound containing a nitrile oxide group and an ionic functional group with a polymer. Use of the ionic functional group-containing polymer improves tire performances such as wet grip performance.

Conventionally, in production of a rubber composition using an ionic bond, the types of ion-modified rubbers are limited. For example, when SBR is used, the SBR skeleton is limited. In contrast, when the nitrile oxide compound is used, it is possible to impart an ionic functional group without depending on the structure of the skeleton, which presumably facilitates adjustment of the desired wet grip performance to be imparted, such as further improvement of the wet grip performance.

The amount of the ionic functional group-containing rubber in 100% by mass of the rubber component in the rubber composition is preferably 5% by mass or more, more preferably 10% by mass or more, still more preferably 150 by mass or more. The lower limit of the amount of the ionic functional group-containing rubber may be 30% by mass or more, 50% by mass or more, 60% by mass or more, or 70% by mass or more. The upper limit is not limited, and may be 100% by mass, 90% by mass or less, 85% by mass or less, or 80% by mass or less. When the amount is within the range indicated above, tire performances such as wet grip performance tend to be better obtained.

When the rubber composition contains the ionic functional group-containing SBR, the amount of the ionic functional group-containing SBR in 100% by mass of the rubber component is preferably 5% by mass or more, more preferably 10% by mass or more, still more preferably 150 by mass or more. The lower limit of the amount of the ionic functional group-containing rubber may be 30% by mass or more, 50% by mass or more, 60% by mass or more, or 70% by mass or more. The upper limit is not limited, and may be 100% by mass, 90% by mass or less, 85% by mass or less, or 80% by mass or less. When the amount is within the range indicated above, tire performances such as wet grip performance tend to be better obtained.

When the rubber composition contains the ionic functional group-containing BR, the amount of the ionic functional group-containing SBR in 100% by mass of the rubber component is preferably 5% by mass or more, more preferably 10% by mass or more, still more preferably 150 by mass or more. The lower limit of the amount of the ionic functional group-containing rubber may be 30% by mass or more, 50% by mass or more, 60% by mass or more, or 70% by mass or more. The upper limit is not limited, and may be 100% by mass, 90% by mass or less, 85% by mass or less, or 80% by mass or less. When the amount is within the range indicated above, tire performances such as wet grip performance tend to be better obtained.

When the rubber composition contains the ionic functional group-containing isoprene-based rubber, the amount of the ionic functional group-containing SBR in 100% by mass of the rubber component is preferably 5% by mass or more, more preferably 10% by mass or more, still more preferably 15% by mass or more. The lower limit of the amount of the ionic functional group-containing rubber may be 30% by mass or more, 50% by mass or more, 60% by mass or more, or 70% by mass or more. The upper limit is not limited, and may be 100% by mass, 90% by mass or less, 850 by mass or less, or 80% by mass or less. When the amount is within the range indicated above, tire performances such as wet grip performance tend to be better obtained.

To better obtain tire performances such as wet grip performance, the rubber composition desirably contains at least one selected from the group consisting of alkali metal salt fillers and alkaline earth metal salt fillers.

The alkali metal salt or alkaline earth metal salt may include, for example, at least one alkali metal salt or alkaline earth metal salt selected from the group consisting of lithium carbonate, sodium carbonate, potassium carbonate, rubidium carbonate, cesium carbonate, beryllium carbonate, magnesium carbonate, calcium carbonate, strontium carbonate, barium carbonate, lithium acetate, sodium acetate, potassium acetate, rubidium acetate, cesium acetate, beryllium

acetate, magnesium acetate, calcium acetate, strontium acetate, barium acetate, lithium phenoxide, sodium phenoxide, potassium phenoxide, rubidium phenoxide, cesium phenoxide, beryllium diphenoxide, magnesium diphenoxide, calcium diphenoxide, strontium diphenoxide, and barium diphenoxide. These alkali metal salts or alkaline earth metal salts may be used alone or in combination of two or more.

To achieve the effect more suitably, the alkali metal salt or alkaline earth metal salt more preferably includes at least one selected from the group consisting of potassium acetate, calcium acetate, sodium acetate, and magnesium acetate, still more preferably includes at least one selected from the group consisting of potassium acetate, calcium acetate, and sodium acetate, particularly preferably includes potassium acetate and/or calcium acetate.

The amount of the alkali metal salt or alkaline earth metal salt (the total amount of the alkali metal salt or alkaline earth metal salt) based on 100 parts by mass of the rubber component in the rubber composition is preferably 0.5 parts by mass or more, more preferably 2.0 parts by mass or more, still more preferably 5.0 parts by mass or more, further still more preferably 7.0 parts by mass or more, particularly preferably 7.2 parts by mass or more, while it is preferably 20.0 parts by mass or less, more preferably 17.0 parts by mass or less, still more preferably 12.0 parts by mass or less, particularly preferably 10.0 parts by mass or less. When the amount is within the range indicated above, the effect tends to be better achieved.

The apparent specific gravity of the alkali metal salt or alkaline earth metal salt is preferably less than 0.4 g/mL, more preferably 0.3 g/mL or less, still more preferably 0.25 g/mL or less, while it is preferably 0.05 g/mL or more, more preferably 0.15 g/mL or more. When the apparent specific gravity is within the range indicated above, the effect tends to be better achieved.

The apparent specific gravity of the alkali metal salt or alkaline earth metal salt is a value calculated from the mass of the alkali metal salt or alkaline earth metal salt in an amount of 30 mL in apparent volume measured into a 50-mL measuring cylinder.

The d50 of the alkali metal salt or alkaline earth metal salt is preferably less than 10 um, more preferably 4.5 um or less, still more preferably 1.5 um or less, particularly preferably less than 0.75 um, while it is preferably 0.05 um or more, more preferably 0.45 um or more. When the d50 is within the range indicated above, the effect tends to be better achieved.

The d50 of the alkali metal salt or alkaline earth metal salt is the particle diameter at the 50th percentile in the mass-based particle size distribution curve obtained by the laser diffraction scattering method.

The nitrogen adsorption specific surface area ($N_2SA$) of the alkali metal salt or alkaline earth metal salt is preferably 100 $m^2/g$ or more, more preferably 115 $m^2/g$ or more, while it is preferably 250 $m^2/g$ or less, more preferably 225 $m^2/g$ or less, still more preferably 200 $m^2/g$ or less. When the $N_2SA$ is within the range indicated above, the effect tends to be better achieved.

The $N_2SA$ of the alkali metal salt or alkaline earth metal salt is a value measured by the BET method according to JIS Z8830:2013.

Usable commercial alkali metal salts or alkaline earth metal salts are available from Kyowa Chemical Corporation, FUJIFILM Wako Pure Chemical Industries, Ltd., Kishida Chemical Co., Ltd., Kyowa Chemical Industry Co., Ltd., Tateho Chemical Industries Co., Ltd., JHE Co., Ltd., Nippon Chemical Industrial Co., Ltd., and Ako Kasei Co., Ltd.

The rubber composition preferably contains a filler. Usable examples of the filler include fillers known in the rubber field, including inorganic fillers such as silica, carbon black, calcium carbonate, talc, alumina, clay, aluminum hydroxide, aluminum oxide, and mica; and difficult-to-disperse fillers. Preferred among these are silica and carbon black, and more preferred is silica.

The total amount of fillers per 100 parts by mass of the rubber component in the rubber composition is preferably 5 parts by mass or more, more preferably 10 parts by mass or more, still more preferably 15 parts by mass or more, particularly preferably 25 parts by mass or more. The upper limit of the total amount is preferably 200 parts by mass or less, more preferably 180 parts by mass or less, still more preferably 160 parts by mass or less. When the total amount is within the range indicated above, tire performances such as wet grip performance tend to be better obtained.

Examples of usable silica include dry silica (anhydrous silica) and wet silica (hydrous silica). Wet silica is preferred among these because it contains a large number of silanol groups. Usable silica may be commercially available from Degussa, Rhodia, Tosoh Silica Corporation, Solvay Japan, Tokuyama Corporation, etc.

The nitrogen adsorption specific surface area ($N_2SA$) of the silica is preferably 10 $m^2/g$ or more, more preferably 20 $m^2/g$ or more, still more preferably 30 $m^2/g$ or more. The upper limit of the $N_2SA$ of the silica is not limited, and it is preferably 300 $m^2/g$ or less, more preferably 275 $m^2/g$ or less, still more preferably 250 $m^2/g$ or less. When the $N_2SA$ is within the range indicated above, tire performances such as wet grip performance tend to be better obtained.

Herein, the $N_2SA$ of silica is measured by the BET method according to ASTM D3037-93.

The amount of the silica per 100 parts by mass of the rubber component in the rubber composition is preferably 5 parts by mass or more, more preferably 10 parts by mass or more, still more preferably 15 parts by mass or more, particularly preferably 25 parts by mass or more. The upper limit of the amount is preferably 200 parts by mass or less, more preferably 180 parts by mass or less, still more preferably 160 parts by mass or less. When the amount is within

the range indicated above, tire performances such as wet grip performance tend to be better obtained.

The rubber composition which contains silica may contain a silane coupling agent together with the silica.

Non-limiting examples of usable silane coupling agents include silane coupling agents conventionally used with silica in the rubber industry, including: sulfide silane coupling agents such as bis(3-triethoxysilylpropyl)tetrasulfide, bis(2-triethoxysilylethyl)tetrasulfide, bis(4-triethoxysilylbutyl)tetrasulfide, bis(3-trimethoxysilylpropyl)tetrasulfide, bis(2-trimethoxysilylethyl)tetrasulfide, bis(2-triethoxysilylethyl)trisulfide, bis(4-trimethoxysilylbutyl)trisulfide, bis(3-triethoxysilylpropyl)disulfide, bis(2-triethoxysilylethyl)disulfide, bis(4-triethoxysilylbutyl)disulfide, bis(3-trimethoxysilylpropyl)disulfide, bis(2-trimethoxysilylethyl)disulfide, bis(4-trimethoxysilylbutyl)disulfide, 3-trimethoxysilylpropyl-N,N-dimethylthiocarbamoyl tetrasulfide, 2-triethoxysilylethyl-N,N-dimethylthiocarbamoyl tetrasulfide, and 3-triethoxysilylpropyl methacrylate monosulfide; mercapto silane coupling agents such as 3-mercaptopropyltrimethoxysilane, 2-mercaptoethyltriethoxysilane, and NXT and NXT-Z both available from Momentive; vinyl silane coupling agents such as vinyltriethoxysilane and vinyltrimethoxysilane; amino silane coupling agents such as 3-aminopropyltriethoxysilane and 3-aminopropyltrimethoxysilane; glycidoxy silane coupling agents such as γ-glycidoxypropyltriethoxysilane and γ-glycidoxypropyltrimethoxysilane; nitro silane coupling agents such as 3-nitropropyltrimethoxysilane and 3-nitropropyltriethoxysilane; and chloro silane coupling agents such as 3-chloropropyltrimethoxysilane and 3-chloropropyltriethoxysilane. Usable commercial products may be available from Degussa, Momentive, Shin-Etsu Silicone, Tokyo Chemical Industry Co., Ltd., AZmax. Co., Dow Corning Toray Co., Ltd., etc. These may be used alone or in combination of two or more. Sulfide silane coupling agents and mercapto silane coupling agents are preferred among these.

The amount of silane coupling agents per 100 parts by mass of silica in the rubber composition is preferably 3 parts by mass or more, more preferably 6 parts by mass or more. The amount is preferably 20 mass parts or less, more preferably 15 mass parts or less.

Examples of usable carbon black include N134, N110, N220, N234, N219, N339, N330, N326, N351, N550, and N762. These may be used alone or in combination of two or more. Usable carbon black may be commercially available from Asahi Carbon Co., Ltd., Cabot Japan K.K., Tokai Carbon Co., Ltd., Mitsubishi Chemical Corporation, Lion Corporation, NSCC Carbon Co., Ltd., Columbia Carbon, etc.

The nitrogen adsorption specific surface area ($N_2SA$) of the carbon black is preferably 10 $m^2/g$ or more, more preferably 20 $m^2/g$ or more, still more preferably 30 $m^2/g$ or more. The upper limit of the $N_2SA$ of the silica is not limited, and is preferably 300 $m^2/g$ or less, more preferably 275 $m^2/g$ or less, still more preferably 250 $m^2/g$ or less. When the $N_2SA$ is within the range indicated above, tire performances such as wet grip performance tend to be better obtained.

Herein, the nitrogen adsorption specific surface area of carbon black can be determined in accordance with JIS K6217-2:2001.

The amount of the carbon black per 100 parts by mass of the rubber component in the rubber composition is preferably 5 parts by mass or more, more preferably 10 parts by mass or more, still more preferably 15 parts by mass or more. The upper limit of the amount is preferably 200 parts by mass or less, more preferably 180 parts by mass or less, still more preferably 160 parts by mass or less. When the amount is within the range indicated above, tire performances such as wet grip performance tend to be better obtained.

The rubber composition may contain a plasticizer. Herein, the term "plasticizer" refers to a material that imparts plasticity to rubber components. Examples include liquid plasticizers (plasticizers which are liquid at room temperature (25°C)) and resins (resins which are solid at room temperature (25°C)).

The amount of plasticizers (total amount of plasticizers) per 100 parts by mass of the rubber component in the rubber composition is preferably 3 parts by mass or more, more preferably 5 parts by mass or more, still more preferably 7 parts by mass or more. The upper limit of the amount is preferably 120 parts by mass or less, more preferably 100 parts by mass or less, still more preferably 90 parts by mass or less. When the amount is within the range indicated above, tire performances such as wet grip performance tend to be better obtained.

Liquid plasticizers (plasticizers which are liquid at room temperature (25°C)) usable in the rubber composition are not limited, and examples include oils and liquid polymers such as liquid resins, liquid diene-based polymers, and liquid farnesene-based polymers. These may be used alone or in combination of two or more.

The amount of liquid plasticizers, i.e., the total amount of plasticizers, per 100 parts by mass of the rubber component in the rubber composition is preferably 3 parts by mass or more, more preferably 5 parts by mass or more, still more preferably 7 parts by mass or more. The upper limit of the amount is preferably 120 parts by mass or less, more preferably 100 parts by mass or less, still more preferably 90 parts by mass or less. When the amount is within the range indicated above, tire performances such as wet grip performance tend to be better obtained. The amount of the liquid plasticizers includes the amount of oils in oil-extended rubbers.

Examples of the oils include process oils, plant oils, and mixtures thereof. Examples of the process oils include paraffinic process oils, aromatic process oils, and naphthenic process oils. Examples of the plant oils include castor oil, cotton seed oil, linseed oil, rapeseed oil, soybean oil, palm oil, coconut oil, peanut oil, rosin, pine oil, pine tar, tall oil, corn oil, rice oil, safflower oil, sesame oil, olive oil, sunflower oil, palm kernel oil, camellia oil, jojoba oil, macadamia nut oil, and tung oil. Usable commercial products may be available from Idemitsu Kosan Co., Ltd., Sankyo Yuka Kogyo K.

K., ENEOS Corporation, Olisoy, H&R, Hokoku Corporation, Showa Shell Sekiyu K.K., Fuji Kosan Co., Ltd., The Nisshin Oillio Group, etc. Process oils such as paraffinic process oils, aromatic process oils, and naphthenic process oils, and plant oils are preferred among these.

Examples of the liquid resins include terpene resins (including terpene phenolic resins and aromatic modified terpene resins), rosin resins, styrene resins, C5 resins, C9 resins, C5/C9 resins, dicyclopentadiene (DCPD) resins, coumarone-indene resins (including resins based on coumarone or indene alone), phenolic resins, olefin resins, polyurethane resins, and acrylic resins. Hydrogenated products of these resins are also usable.

Examples of the liquid diene-based polymers include liquid styrene-butadiene copolymers (liquid SBR), liquid butadiene polymers (liquid BR), liquid isoprene polymers (liquid IR), liquid styrene-isoprene copolymers (liquid SIR), liquid styrene-butadiene-styrene block copolymers (liquid SBS block polymers), liquid styrene-isoprene-styrene block copolymers (liquid SIS block polymers), liquid farnesene polymers, and liquid farnesene-butadiene copolymers, all of which are liquid at 25°C. The chain ends or backbones of these polymers may be modified with a polar group. Hydrogenated products of these polymers are also usable.

Examples of the resin (resin which is solid at room temperature (25°C)) usable in the rubber composition include aromatic vinyl polymers, coumarone-indene resins, coumarone resins, indene resins, phenolic resins, rosin resins, petroleum resins, terpene-based resins, and acrylic resins, all of which are solid at room temperature (25°C). Also, the resin may be hydrogenated. These may be used alone or in combination of two or more. Preferred among these are aromatic vinyl polymers, petroleum resins, and terpene resins.

The amount of the resin per 100 parts by mass of the rubber component in the rubber composition is preferably 3 parts by mass or more, more preferably 5 parts by mass or more, still more preferably 7 parts by mass or more. The upper limit of the amount is preferably 120 parts by mass or less, more preferably 100 parts by mass or less, still more preferably 90 parts by mass or less. When the amount is within the range indicated above, tire performances such as wet grip performance tend to be better obtained.

The softening point of the resin is preferably 50°C or higher, more preferably 55°C or higher, still more preferably 60°C or higher. The upper limit of the softening point is preferably 160°C or lower, more preferably 150°C or lower, still more preferably 145°C or lower. When the softening point is within the range indicated above, tire performances such as wet grip performance tend to be better obtained. Herein, the softening point of the resin is determined in accordance with JIS K 6220-1:2001 using a ring and ball softening point measuring apparatus and defined as the temperature at which the ball drops down.

The term "aromatic vinyl polymers" refers to polymers containing aromatic vinyl monomers as structural units. Examples thereof include resins obtainable by polymerizing α-methylstyrene and/or styrene. Specific examples include styrene homopolymers (styrene resins), α-methylstyrene homopolymers (a-methylstyrene resins), copolymers of α-methylstyrene and styrene, and copolymers of styrene and other monomers.

The term "coumarone-indene resins" refers to resins containing coumarone and indene as the monomer components mainly forming the skeleton (backbone) of the resins. Examples of monomer components which may be contained in the skeleton in addition to coumarone and indene include styrene, α-methylstyrene, methylindene, and vinyltoluene.

The term "coumarone resins" refers to resins containing coumarone as a monomer component mainly forming the skeleton (backbone) of the resins.

The indene resins refer to resins containing indene as a monomer component mainly forming the skeleton (backbone) of the resins.

The phenolic resins used may be known resins such as polymers obtained by reacting phenol with an aldehyde such as formaldehyde, acetaldehyde, or furfural in the presence of an acid or alkali catalyst. Preferred among these are phenolic resins obtained by reaction in the presence of an acid catalyst (such as novolac phenolic resins).

Examples of the rosin resin include rosin-based resins typified by natural rosins, polymerized rosins, modified rosins, esterified compounds of these rosins, and hydrogenated products of these rosins.

Examples of the petroleum resins include C5-based resins, C9-based resins, C5/C9-based resins, dicyclopentadiene (DCPD) resins, and hydrogenated products thereof. Preferred among these are DCPD resins and hydrogenated DCPD resins.

The term "terpene-based resins" refers to polymers containing terpene as a structural unit. Examples thereof include polyterpene resins obtainable by polymerizing terpene compounds, and aromatic modified terpene resins obtainable by polymerizing terpene compounds and aromatic compounds. Examples of the aromatic modified terpene resins include terpene-phenolic resins made from terpene compounds and phenolic compounds, terpene-styrene resins made from terpene compounds and styrene compounds, and terpene-phenol-styrene compounds made from terpene compounds, phenolic compounds, and styrene compounds. Examples of the terpene compounds include α-pinene and β-pinene. Examples of the phenolic compounds include phenol and bisphenol A. Examples of the aromatic compounds include styrene compounds (styrene, α-methylstyrene, etc.).

The term "acrylic resins" refer to polymers containing acrylic monomers as structural units. Examples include

styrene-acrylic resins that contain carboxy groups and are obtained by copolymerizing aromatic vinyl monomer components and acrylic monomer components, such as a styrene acrylic resin. In particular, solvent-free carboxy group-containing styrene-acrylic resins are suitably usable.

Usable commercial plasticizers may be available from Maruzen Petrochemical Co., Ltd., Sumitomo Bakelite Co., Ltd., Yasuhara Chemical Co., Ltd., Tosoh Corporation, Rutgers Chemicals, BASF, Arizona Chemical, Nitto Chemical Co., Ltd., Nippon Shokubai Co., Ltd., ENEOS Corporation, Arakawa Chemical Industries, Ltd., Taoka Chemical Co., Ltd., etc.

From the standpoint of properties such as cracking resistance and ozone resistance, the rubber composition preferably contains an antioxidant.

Any antioxidant may be used. Examples include naphthylamine antioxidants such as phenyl-$\alpha$-naphthylamine; diphenylamine antioxidants such as octylated diphenylamine and 4,4'-bis($\alpha$,$\alpha$'-dimethylbenzyl)diphenylamine; p-phenylenediamine antioxidants such as N-isopropyl-N'-phenyl-p-phenylenediamine, N-(1,3-dimethylbutyl)-N'-phenyl-p-phenylenediamine, and N,N'-di-2-naphthyl-p-phenylenediamine; quinoline antioxidants such as polymerized 2,2,4-trimethyl-1,2-dihydroquinoline; monophenolic antioxidants such as 2,6-di-t-butyl-4-methylphenol and styrenated phenol; and bis-, tris-, or polyphenolic antioxidants such as tetrakis[methylene-3-(3',5'-di-t-butyl-4'-hydroxyphenyl)propionate]methane. Preferred among these are p-phenylenediamine or quinoline antioxidants, and N-(1,3-dimethylbutyl)-N'-phenyl-p-phenylenediamine or polymerized 2,2,4-trimethyl-1,2-dihydroquinoline is more preferred. Usable commercial products may be available from Seiko Chemical Co., Ltd., Sumitomo Chemical Co., Ltd., Ouchi Shinko Chemical Industrial Co., Ltd., Flexsys, etc.

The amount of antioxidants per 100 parts by mass of the rubber component in the rubber composition is preferably 0.2 parts by mass or more, more preferably 0.5 parts by mass or more. The amount is preferably 7.0 parts by mass or less, more preferably 4.0 parts by mass or less.

The rubber composition may contain stearic acid. The amount of stearic acid per 100 parts by mass of the rubber component in the rubber composition is preferably 0.5 to 10 parts by mass, more preferably 0.5 to 5 parts by mass.

The stearic acid may be conventional ones. Usable commercial products may be available from NOF Corporation, Kao Corporation, FUJIFILM Wako Pure Chemical Corporation, Chiba Fatty Acid Co., Ltd., etc.

The rubber composition may contain zinc oxide. The amount of zinc oxide per 100 parts by mass of the rubber component in the rubber composition is preferably 0.5 to 10 parts by mass, more preferably 1 to 5 parts by mass.

The zinc oxide may be conventional ones. Usable commercial products may be available from Mitsui Mining & Smelting Co., Ltd., Toho Zinc Co., Ltd., HakusuiTech Co., Ltd., Seido Chemical Industry Co., Ltd., Sakai Chemical Industry Co., Ltd., etc.

The rubber composition may contain wax. The amount of wax per 100 parts by mass of the rubber component in the rubber composition is preferably 0.5 to 10 parts by mass, more preferably 1 to 5 parts by mass.

Any wax may be used, and examples include petroleum waxes and natural waxes and also include synthetic waxes prepared by purifying or chemically treating a plurality of waxes. These waxes may be used alone or in combination of two or more.

Examples of the petroleum waxes include paraffin waxes and microcrystalline waxes. The natural waxes may be any wax derived from non-petroleum resources, and examples include plant waxes such as candelilla wax, carnauba wax, Japan wax, rice wax, and jojoba wax; animal waxes such as beeswax, lanolin, and spermaceti; mineral waxes such as ozokerite, ceresin, and petrolatum; and purified products of these waxes. Usable commercial products may be available from Ouchi Shinko Chemical Industrial Co., Ltd., Nippon Seiro Co., Ltd., Seiko Chemical Co., Ltd., etc.

The rubber composition may contain sulfur to moderately form a crosslinked chain between the polymer chains, thereby achieving a good balance among the performances.

The amount of sulfur per 100 parts by mass of the rubber component in the rubber composition is preferably 0.1 parts by mass or more, more preferably 0.5 parts by mass or more, still more preferably 0.7 parts by mass or more. The amount is preferably 6.0 parts by mass or less, more preferably 4.0 parts by mass or less, still more preferably 3.0 parts by mass or less.

Examples of the sulfur include those commonly used in the rubber industry, such as powdered sulfur, precipitated sulfur, colloidal sulfur, insoluble sulfur, highly dispersible sulfur, and soluble sulfur. Usable commercial products may be available from Tsurumi Chemical Industry Co., Ltd., Karuizawa sulfur Co., Ltd., Shikoku Chemicals Corporation, Flexsys, Nippon Kanryu Industry Co., Ltd., Hosoi Chemical Industry Co., Ltd., etc. These may be used alone or in combination of two or more.

The rubber composition may contain a vulcanization accelerator.

The amount of vulcanization accelerators per 100 parts by mass of the rubber component in the rubber composition is normally 0.3 to 10 parts by mass, preferably 0.5 to 7 parts by mass.

Any type of vulcanization accelerator may be used, including usually used ones. Examples of vulcanization accelerators include thiazole vulcanization accelerators such as 2-mercaptobenzothiazole, di-2-benzothiazolyl disulfide, and N-cyclohexyl-2-benzothiazylsulfenamide; thiuram vulcanization accelerators such as tetramethylthiuram disulfide

(TMTD), tetrabenzylthiuram disulfide (TBzTD), and tetrakis(2-ethylhexyl)thiuram disulfide (TOT-N); sulfenamide vulcanization accelerators such as N-cyclohexyl-2-benzothiazole sulfenamide, N-t-butyl-2-benzothiazolylsulfenamide, N-oxyethylene-2-benzothiazole sulfenamide, and N,N'-diisopropyl-2-benzothiazole sulfenamide; and guanidine vulcanization accelerators such as diphenylguanidine, diorthotolylguanidine, and orthotolylbiguanidine. These may be used alone or in combination of two or more. Sulfenamide vulcanization accelerators and guanidine vulcanization accelerators are preferred among these.

The rubber composition may contain other appropriate additives usually used in the application field, such as a release agent or a pigment, in addition to the above-described components.

The rubber composition may be prepared by known methods. For example, it may be prepared for example by kneading the components in a rubber kneading machine such as an open roll mill or a Banbury mixer, optionally followed by crosslinking. The kneading conditions include a kneading temperature of usually 50°C to 200°C, preferably 80°C to 190°C, and a kneading time of usually 30 seconds to 30 minutes, preferably 1 minute to 30 minutes.

The rubber composition is usable in tires, footwear soles, floor materials, vibration-proof materials, seismic isolators, butyl frame materials, belts, hoses, packing materials, medical stoppers, and other industrial rubber products. In particular, the rubber composition is preferably used as a rubber composition for tires due to its excellent tire performances such as wet grip performance.

To obtain better tire performances such as wet grip performance, the rubber composition preferably satisfies the following relationship (1).

$$(1)\ E^{*}\ \text{when wet with water}/E^{*}\ \text{when dry} \leq 0.95$$

In the relationship, E* refers to a complex modulus of elasticity (MPa) 30 minutes after the start of measurement at a temperature of 30°C, an initial strain of 10%, a dynamic strain of 10, and a frequency of 10 Hz in an elongation mode for a measurement time of 30 minutes.

The ratio: E* when wet with water/E* when dry is preferably 0.91 or lower, more preferably 0.88 or lower, still more preferably 0.86 or lower, particularly preferably 0.84 or lower, most preferably 0.81 or lower. The lower limit of the ratio: E* when wet with water/E* when dry is not limited, and it is preferably 0.10 or higher, more preferably 0.30 or higher, still more preferably 0.50 or higher, particularly preferably 0.60 or higher. When the ratio is within the range indicated above, the effect can be suitably achieved.

The rubber composition preferably has an E* when dry of 3.0 MPa or more, more preferably 52.0 MPa or more, still more preferably 58.0 MPa or more, particularly preferably 66.0 MPa or more. The upper limit of the E* when dry is not limited, and it is preferably 200.0 MPa or less, more preferably 176.0 MPa or less, still more preferably 150.0 MPa or less, particularly preferably 100.0 MPa or less. When the E* when dry is within the range indicated above, the effect can be suitably achieved.

The rubber composition preferably has an E* when wet with water of 3.0 MPa or more, more preferably 45.0 MPa or more, still more preferably 55.0 MPa or more, particularly preferably 60.0 MPa or more. The upper limit of the E* when dry is not limited, and it is preferably 180.0 MPa or less, more preferably 150.0 MPa or less, still more preferably 110.0 MPa or less, particularly preferably 90.0 MPa or less. When the E* when wet with water is within the range indicated above, the effect can be suitably achieved.

Herein, the complex modulus of elasticity (E*) of a rubber composition refers to the E* of the vulcanized rubber composition. The E* is a value determined by a viscoelastic test of the vulcanized rubber composition.

The rubber composition satisfies the relationship (1) and reversibly changes its complex modulus of elasticity (E*) with water, for example. Herein, the expression "reversibly changes its complex modulus of elasticity (E*) with water" means that the E* of the (vulcanized) rubber composition reversibly increases or decreases depending on the presence of water. It is sufficient that the E* reversibly changes when the state of the rubber composition changes as follows: dry → wet with water → dry, for example. The rubber composition in the former dry state may or may not have the same E* as in the latter dry state.

Herein, the term "E* when dry" means the E* of the rubber composition which is dry and specifically refers to the E* of the rubber composition which has been dried as described in EXAMPLES.

Herein, the term "E* when wet with water" means the E* of the rubber composition which is wet with water and specifically refers to the E* of the rubber composition which has been wet with water as described in EXAMPLES.

Herein, the E* of the rubber composition refers to E* 30 minutes after the start of measurement at a temperature of 30°C, an initial strain of 10%, a dynamic strain of 10, and a frequency of 10 Hz in an elongation mode for a measurement time of 30 minutes.

The reversible E* change with water of the rubber composition expressed by the relationship (1) may be achieved, for example, by allowing the rubber composition to contain the ionic functional group-containing polymer and, if necessary, the alkali metal salt or alkaline earth metal salt.

The E* when dry can be controlled by the types and amounts of chemicals (in particular, rubber components, fillers, and softeners such as oils) blended in the rubber composition. For example, the E* when dry tends to be increased by reducing the amount of softeners or increasing the amount of fillers.

The E* when dry can be controlled, for example, by the ionic functional group content of the ionic functional group-containing polymer and the amount of the alkali metal salt or alkaline earth metal salt (in other words, the metal content derived from the alkali metal salt or alkaline earth metal salt). Specifically, the E* when dry tends to be increased by increasing the ionic functional group content of the ionic functional group-containing polymer or the amount of the alkali metal salt or alkaline earth metal salt.

The E* when wet with water can be controlled to be lower than the E* when dry by, for example, allowing the ionic functional group-containing polymer and the alkali metal salt or alkaline earth metal salt in the rubber composition to be partly or entirely crosslinked by ionic bonds. Thus, the E* when wet with water and the E* when dry can be controlled. Specifically, when the ionic functional group-containing polymer and the alkali metal salt or alkaline earth metal salt are used in combination, the rubber composition crosslinked by ionic bonds is obtained. Thus, the E* when wet with water can be lower than the E* when dry. The E* when wet with water can be controlled by the types and amounts of the chemicals blended in the rubber composition. For example, the E* when wet with water can have the same tendency as described above by using the same techniques as those for controlling the E* when dry.

Specifically, the rubber composition can achieve the reversible E* change with water expressed by the relationship (1), for example, when it contains the ionic functional group-containing polymer and, if necessary, the alkali metal salt or alkaline earth metal salt, while controlling the E* when dry to be within the desired range.

The rubber composition may be used in any tire component. Examples of the tire component include cap treads, sidewalls, base treads, bead apexes, clinch apexes, innerliners, undertreads, breaker toppings, and ply toppings. In particular, the rubber composition is suitable for cap treads because of its excellent wet grip performance, etc.

<Tire>

The rubber composition is suitable for use in a tire. Examples of the tire include pneumatic tires and non-pneumatic tires. Pneumatic tires are preferred among these. In particular, the rubber composition may be suitably used in summer tires, winter tires (e.g., studless tires, snow tires, or studded tires), all-season tires, for example. The tire may be used as a tire for passenger vehicles, large passenger vehicles, large SUVs, heavy duty vehicles such as trucks and buses, light trucks, or motorcycles, or as a racing tire (high performance tire), for example. The tire is especially suitable for passenger vehicles or light trucks.

The tire is produced using the rubber composition by usual methods. For example, the rubber composition containing the materials, before vulcanization, may be extruded into the shape of a tire component and assembled with other tire components on a tire building machine in a usual manner to build an unvulcanized tire. The unvulcanized tire is heated and pressurized in a vulcanizer, whereby the tire can be produced.

EXAMPLES

The present invention will be specifically described based on examples, but the present invention is not limited thereto.

<Synthesis 1 of nitrile oxide compound>

A compound E (a nitrile oxide compound containing a nitrile oxide group and an ionic functional group) was synthesized according to the synthetic route shown in FIG. 1.

(Compound A → Compound B)

First, a three-necked flask was charged with a stirring bar, 2-hydroxy-1-naphthaldehyde (compound A), $K_2CO_3$, N,N-dimethylformamide (DMF), and ethyl 6-bromohexanoate, and the contents were dissolved by stirring. The resulting solution was reacted by heating in an oil bath at 100°C for three hours. After cooling to room temperature, the solution was separated using ethyl acetate and water. The obtained ethyl acetate layer was dried using $MgSO_4$, followed by evaporation. The residue was recrystallized using hexane, whereby a compound B was obtained.

(Compound B -> Compound C)

The compound B and NaOH were dissolved in a solvent mixture of THF:iPrOH:$H_2O$ = (4:2:1) and allowed to react at room temperature overnight. After completion of the reaction, the solution was neutralized using hydrochloric

acid, followed by evaporation. The residue was diluted with water and put into a separatory funnel together with ethyl acetate, whereby the layers were separated. The obtained ethyl acetate layer was dried using $MgSO_4$, evaporated to be dried, and further dried in vacuo, whereby a compound C was obtained.

(Compound C -> Compound D)

A 500-ml beaker was charged with a stirring bar, the compound C, and ethanol, and the contents were dissolved by stirring. An aqueous solution of $CH_3COONa{\cdot}3H_2O$ and $NH_2OH{\cdot}HCl$ was added to the compound C solution and reacted at room temperature for four hours, whereby a precipitate was formed. The mixture was filtered to collect the precipitate. The precipitate was dried in vacuo, whereby a compound D was obtained.

(Compound D → Compound E)

A three-necked flask was charged with a stirring bar, the compound D, and 2-propanol, and placed in an ice bath, followed by dispersion of the contents by stirring. Under the condition of 0°C to 5°C, a NaClO solution was added to the flask through a dropping funnel and the contents were reacted. After completion of the reaction, the pH of the reaction solution was adjusted to about 5 using HCl, followed by separation using $CHCl_3$. The obtained $CHCl_3$ layer was dried using $MgSO_4$, evaporated to be further dried, and recrystallized using ethanol/water, whereby a target compound E (nitrile oxide compound containing a nitrile oxide group and an ionic functional group) was obtained.

The $^1$H-NMR spectrum (FIG. 2) and IR spectrum (FIG. 3) of the synthesized product confirmed the synthesis of the compound E (nitrile oxide compound containing a nitrile oxide group and an ionic functional group).

<Synthesis 2 of nitrile oxide compound>

A compound H (a nitrile oxide compound containing a nitrile oxide group and an ionic functional group) was synthesized according to the synthetic route shown in FIG. 6.

(Compound A → Compound F)

First, a three-necked flask was charged with a stirring bar, 2-hydroxy-1-naphthaldehyde (compound A), $K_2CO_3$, N,N-dimethylformamide (DMF), and 6-chlorohexanol, and the contents were dissolved by stirring. The solution was reacted by heating in an oil bath at 100°C for five hours. After cooling to room temperature, the resulting solution was separated using ethyl acetate and water. The obtained ethyl acetate layer was dried using $MgSO_4$ and evaporated, whereby a compound F was obtained.

(Compound F → Compound G)

A 500-ml beaker was charged with a stirring bar, the compound F, and ethanol, and the contents were dissolved by stirring. An aqueous solution of $CH_3COONa{\cdot}3H_2O$ and $NH_2OH{\cdot}HCl$ was added to the compound F solution and reacted at room temperature for four hours, whereby a precipitate was formed. The mixture was filtered to collect the precipitate. The precipitate was dried in vacuo, whereby a compound G was obtained.

(Compound G -> Compound H)

A three-necked flask was charged with a stirring bar, the compound G, and 2-propanol, and placed in an ice bath, followed by dispersion of the contents by stirring. Under the condition of 0°C to 5°C, a NaClO solution was added to the flask through a dropping funnel and the contents were reacted. After completion of the reaction, the pH of the reaction solution was adjusted to about 5 using HCl, followed by precipitation using water and subsequent filtering, whereby a solid target compound H (nitrile oxide compound containing a nitrile oxide group and an ionic functional group) was obtained.

(Compound H → Compound I)

A three-necked flask was charged with a stirring bar, the compound H, and dry THF, and placed in an ice bath, followed by dissolution of the contents by stirring. Under the condition of 0°C to 5°C, a dry THF solution of 2-bromoisobutyrylbromide was added to the flask through a dropping funnel and the contents were reacted. After completion of the reaction, the reaction solution was filtered, and the filtrate was separated using water/ethyl acetate. The obtained organic layer was dried using $MgSO_4$ and evaporated, whereby a compound I was obtained.

The $^1H$-NMR spectrum and IR spectrum of the synthesized product confirmed the synthesis of the compound H (nitrile oxide compound containing a nitrile oxide group and an ionic functional group).

<Synthesis 3 of nitrile oxide compound>

A compound e (nitrile oxide compound containing a nitrile oxide group and an ionic functional group) was synthesized according to the synthetic route shown in FIG. 7.

(Compound a -> Compound b)

A three-necked flask was charged with a stirring bar, the compound a, 4-(bromomethyl) benzoate, $K_2CO_3$, and 18-crown-6, and the contents were dissolved by stirring. The resulting solution was reacted by heating in an oil bath at 40°C overnight. The reaction solution was evaporated, and the solid content was separated using dichloromethane and a 3% hydrochloric acid aqueous solution. The obtained dichloromethane layer was dried using $MgSO_4$ and evaporated. The solid content was recrystallized using hexane/ethyl acetate (7/1 v/v), whereby a compound b was obtained.

(Compound b -> Compound c)

The compound b and LiOH were dissolved in a solvent mixture of THF:iPrOH:$H_2O$ = (4:2:1) and allowed to react at room temperature overnight. After completion of the reaction, the pH of the reaction solution was adjusted to 5 using hydrochloric acid, followed by evaporation of the reaction solution. The residue was separated using dichloromethane/water, and the obtained organic layer was dried using $MgSO_4$ and evaporated, whereby a compound c was obtained.

(Compound c -> Compound d)

A 500-ml beaker was charged with a stirring bar, the compound c, and ethanol, and the contents were dissolved by stirring. An aqueous solution of $CH_3COONa{\cdot}3H_2O$ and $NH_2OH{\cdot}HCl$ was added to the compound c solution and reacted at room temperature for four hours, whereby a precipitate was formed. The mixture was filtered to collect the precipitate. The precipitate was dried in vacuo, whereby a compound d was obtained.

(Compound d -> Compound e)

A three-necked flask was charged with a stirring bar, the compound d, and DMF, and placed in an ice bath, followed by dissolution of the contents by stirring. Under the condition of 0°C to 5°C, a DMF solution of NBS was added to the flask through a dropping funnel and the contents were reacted. After 10 minutes, a DMF solution of TEA was added to the flask through a dropping funnel and the contents were reacted. After completion of the reaction, the reaction solution was separated using water/ethyl acetate, and the obtained organic layer was dried using $MgSO_4$ and evaporated, whereby a compound e (nitrile oxide compound containing a nitrile oxide group and an ionic functional group) was obtained.

The $^1H$-NMR spectrum and IR spectrum of the synthesized product confirmed the synthesis of the compound e (nitrile oxide compound containing a nitrile oxide group and an ionic functional group).

<Synthesis of ionic functional group-containing polymer>

The compound E (nitrile oxide compound containing a nitrile oxide group and an ionic functional group) obtained in the synthesis of nitrile oxide compound and polymers were dissolved in solvents according to the formulations and conditions shown in Table 1 to prepare solutions. The solutions were then reacted by stirring. After completion of the reaction, the solutions containing the resulting products were subjected to precipitation using methanol, and the precipitates were dried, whereby diene-based rubbers containing carboxylic acid groups (ionic functional group-containing polymers (BR1-1 to BR1-5, ESBR-1 to ESBR-9, SSBR1-1 to SSBR1-3), (BR2-1 to BR2-2, SSBR2-1 to SSBR2-2, SSBR1-4 to SSBR1-5)) were obtained.

The double bond modification rate and nitrile oxide compound reaction rate in Table 1 are defined below and measured by the following methods.

(Double bond modification rate (%)) = [Amount of carbon-carbon double bond reacted with nitrile oxide compound/(Amount of remaining carbon-carbon double bond + Amount of carbon-carbon double bond reacted with nitrile oxide compound)] × 100

(Nitrile oxide compound reaction rate (%)) = (Amount of nitrile oxide compound reacted with double bond/(Amount charged of nitrile oxide compound) × 100

(1) Double bond modification rate

The percentage of the nitrile oxide compound added to the carbon-carbon double bond in the polymer was calculated by IR analysis, $^{1}$H-NMR analysis, and $^{13}$C-NMR analysis.

(2) Nitrile oxide compound reaction rate

The nitrile oxide compound reaction rate was determined by IR analysis, $^{1}$H-NMR analysis, and $^{13}$C-NMR analysis.

(Measurement of glass transition temperature (Tg))

The glass transition temperature (Tg) of each ionic functional group-containing polymer produced was determined as the glass transition starting temperature by the measurement in accordance with JIS K 7121 using a differential scanning calorimeter (Q200) available from TA Instruments Japan at a heating rate of 10°C/min.

[Table 1]

| Ionic funtional group-containing polymer | Nitrile oxide compound (g) | Polymer (g) | Condition | | | | | Double bond modification rate | Nitrile oxide compound reaction rate | Tg (°C) |
|---|---|---|---|---|---|---|---|---|---|---|
| | | | Amount charged of nitrile oxide compound (relative to double bond) | Solvent (mL) | Catalyst (mL) | Temperature (°C) | Reaction time (h) | | | |
| BR1-1 | 0.5 | BR1 (0.5) | 18% | $CHCl_3$ (20) | - | 70 | 6 | 0.24% | 1.3% | - |
| BR1-2 | 1 | BR1 (0.5) | 36% | $CHCl_3$ (40) | - | 70 | 30 | 0.54% | 15% | - |
| BR1-3 | 1 | BR1 (0.5) | 36% | $CHCl_3$ (40) | - | Normal temperature | 48 | 0.68% | 1.9% | - |
| BR1-4 | 0.4 | BR1 (0.5) | 14.4% | THF (20) | DMF (1) | 70 | 48 | 2.7 % | 18.8 % | - |
| BR1-5 | 0.5 | BR1 (0.5) | 18% | THF (20) | DMSO (1) | 70 | 48 | 4.6 % | 26.1% | - |
| ESBR-1 | 0.5 | ESBR (0.5) | 25% | $CHCl_3$ (20) | - | 70 | 6 | 0.13% | 0.5% | - |
| ESBR-2 | 0.5 | ESBR (0.5) | 25% | $CHCl_3$ (20) | - | Normal temperature | 48 | 3.7 % | 14.8% | - |
| ESBR-3 | 0.5 | ESBR (0.5) | 25% | $CHCl_3$ (20) | TEA (0.5) | Normal temperature | 72 | 2% | 8% | - |
| ESBR-4 | 0.5 | ESBR (0.5) | 25% | THF (20) | - | Normal temperature | 96 | 6.8 % | 27.2% | - |
| ESBR-5 | 0.5 | ESBR (0.5) | 25% | THF (20) | TEA (0.5) | Normal temperature | 96 | 9.2% | 36.8 % | - |
| ESBR-6 | 1 | ESBR (0.5) | 50% | THF (20) | - | Normal temperature | 96 | 10.7 % | 21.4% | - |
| ESBR-7 | 0.5 | ESBR (0.5) | 25% | THF (20) | DMF (1) | 70 | 48 | 5.3% | 21.2% | - |

(continued)

| Ionic funtional group-containing polymer | Nitrile oxide compound (g) | Polymer (g) | Condition | | | | | Double bond modification rate | Nitrile oxide compound reaction rate | Tg (°C) |
|---|---|---|---|---|---|---|---|---|---|---|
| | | | Amount charged of nitrile oxide compound (relative to double bond) | Solvent (mL) | Catalyst (mL) | Temperature (°C) | Reaction time (h) | | | |
| ESBR-8 | 1 | ESBR (0.5) | 50% | THF (20) | DMF (1) | 70 | 48 | 10.2% | 20.4% | - |
| ESBR-9 | 0.5 | ESBR (0.5) | 25% | THF (20) | DMSO (1) | 70 | 48 | 87% | 34.8 % | - |
| SSBR1-1 | 0.5 | SSBR1 (0.5) | 25% | THF (20) | DMSO (1.5) | 70 | 48 | 7.5 % | 30.0% | - |
| SSBR1-2 | 0.5 | SSBR1 (0.5) | 25% | THF (20) | DMSO (15) | Normal temperature | 48 | 5.7% | 22.8 % | - |
| SSBR1-3 | 1 | SSBR1 (0.5) | 50% | THF (20) | DMSO (15) | Normal temperature | 48 | 10.3% | 20.6% | - |
| BR2-1 | 14.5 | BR2 (26.25) | 10% | THF ( 276 ) | DMSO ( 30 ) | 70 | 48 | 4.5% | 450% | -35 |
| BR2-2 | 8.23 | BR2 (29.90) | 5% | THF ( 316 ) | DMSO ( 34 ) | 70 | 48 | 2.6 % | 520% | -59 |
| SSBR2-1 | 6.92 | SSBR2 (34.8) | 4% | THF ( 420 ) | DMSO ( 35 ) | 70 | 48 | 2.8 % | 670% | -49 |
| SSBR2-2 | 3.46 | SSBR2 (34.8) | 2% | THF ( 420) | DMSO ( 35 ) | 70 | 48 | 1.4% | 670% | -55 |
| SSBR1-4 | 11.4 | SSBR1 (27.9) | 10% | THF ( 335 ) | DMSO ( 29 ) | 70 | 48 | 5.9 % | 59.0% | -2.3 |
| SSBR1-5 | 6.3 | SSBR1 (31.0) | 5% | THF ( 372 ) | DMSO ( 35 ) | 70 | 48 | 30% | 600% | -10.8 |

The following materials were used for BR1, ESBR, SSBR1, BR2, and SSBR2 in Table 1.

BR1: BR150B (cis content 96% by mass) available from Ube Industries, Ltd.

ESBR: Nipol 1502 (E-SBR, styrene content 23.5% by mass, vinyl content 18% by mass) available from ZEON Corporation.

SSBR1: HPR850 (S-SBR) available from JSR Corporation

BR2: BR730 (BR synthesized using a Nd-based catalyst, cis content 96% by mass) available from JSR Corporation

SSBR2: HPR840 (S-SBR) available from JSR Corporation

The $^1$H-NMR spectrum of each synthesized product (FIG. 5) confirmed the synthesis of a diene-based rubber containing a carboxylic acid group (ionic functional group-containing polymer).

From the Tg values of BR2-1, BR2-2, SSBR2-1, SSBR2-2, SSBR1-4, and SSBR1-5 (ionic functional group-containing polymers) in Table 1, it was confirmed that the ionic functional group-containing polymer has a higher Tg than the corresponding polymer.

As described above, it is possible to synthesize a nitrile oxide compound containing a nitrile oxide group and an ionic functional group, and it is also possible to synthesize an ionic functional group-containing polymer by reacting the nitrile oxide compound with BR, ESBR, or SSBR.

Since the nitrile oxide group in a nitrile oxide compound containing a nitrile oxide group and an ionic functional group is highly reactive and can react with any polymer, reacting the nitrile oxide group in the nitrile oxide compound with an arbitrary polymer (e.g., an isoprene-based rubber, a polymer containing a different double bond) presumably can introduce the ionic functional group into the polymer, which is believed to enable provision of polymers containing various ionic functional groups.

<Production of rubber composition>

Various chemicals used below will be collectively described.

SSBR1: HPR850 (S-SBR) available from JSR Corporation
SSBR1-4: ionic functional group-containing SBR produced according to Table 1
SSBR1-5: ionic functional group-containing SBR produced according to Table 1
SSBR2: HPR840 (S-SBR) available from JSR Corporation
SSBR2-1: ionic functional group-containing SBR produced according to Table 1
SSBR2-2: ionic functional group-containing SBR produced according to Table 1
BR2: BR730 (BR synthesized using a Nd-based catalyst, cis content 96% by mass) available from JSR Corporation
BR2-1: ionic functional group-containing BR produced according to Table 1
BR2-2: ionic functional group-containing BR produced according to Table 1
Potassium acetate: potassium acetate available from FUJIFILM Wako Pure Chemical Industries, Ltd.
Silica: Ultrasil VN3 available from Evonik Degussa ($N_2SA$: 175 $m^2/g$)
Silane coupling agent: Si266 available from Evonik Degussa
Stearic acid: stearic acid "Tsubaki" available from NOF Corporation
Oil: Diana Process NH-70S available from Idemitsu Kosan Co., Ltd.
Sulfur: powdered sulfur available from Tsurumi Chemical Industry Co., Ltd.
Vulcanization accelerator NS: NOCCELER NS (N-tert-butyl-2-benzothiazylsulfenamide) available from Ouchi Shinko Chemical Industrial Co., Ltd.
Vulcanization accelerator DPG: NOCCELER D (1,3-diphenylguanidine) available from Ouchi Shinko Chemical Industrial Co., Ltd.

(Production of rubber composition)

According to the formulations in Tables 2 to 5, the materials other than sulfur and vulcanization accelerators were kneaded at 160°C for four minutes using a 16-L Banbury mixer (Kobe Steel, Ltd.), whereby kneaded mixtures were obtained. To the kneaded mixtures were added the sulfur and the vulcanization accelerators, and they were kneaded using an open roll mill at 80°C for four minutes, whereby unvulcanized rubber compositions were obtained.

The resulting unvulcanized rubber compositions were press-vulcanized at 170°C for 12 minutes, whereby

vulcanized rubber compositions were obtained.

The physical properties of the vulcanized rubber compositions thus obtained were measured and evaluated as described below. The results are shown in the tables. The reference comparative examples were as follows.

Table 2: Comparative Example 1-1
Table 3: Comparative Example 2-1
Table 4: Comparative Example 3-1
Table 5: Comparative Example 4-1

<Viscoelastic test>

A viscoelastic test sample having a length of 40 mm, a width of 3 mm, and a thickness of 0.5 mm was collected from each vulcanized rubber composition, and the E* of the vulcanized rubber composition was measured using an RSA series analyzer available from TA Instruments after 30 minutes from the start of the measurement at a temperature of 30°C, an initial strain of 10%, a dynamic strain of 10, and a frequency of 10 Hz in an elongation mode for a measurement time of 30 minutes.

<E* when dry>

The viscoelastic test sample (40 mm in length × 3 mm in width × 0.5 mm in thickness) was dried at room temperature and normal pressure to a constant weight. The complex modulus of elasticity E* of the resulting dried vulcanized rubber composition (rubber piece) was measured by the above-described viscoelastic test method, and the measured E* was determined as E* when dry.

<E* when wet with water>

The viscoelasticity of the sample was measured in water by the above viscoelastic test method using an immersion measurement jig of the RSA, and the measured E* was determined as E* when wet with water. The temperature of the water was 30°C.

<Reduction of E* when wet with water>

The reciprocal of the ratio "E* when wet with water/E* when dry" of each vulcanized rubber composition was expressed as an index relative to the ratio "E* when wet with water/E* when dry" of the vulcanized rubber composition of the reference comparative example taken as 100. A higher index indicates better reduction of E* when wet with water, indicating that the wet performances such as wet grip performance are excellent.

[Table 2]

| | | Comparative Example | Example |
|---|---|---|---|
| | | 1-1 | 1-1 |
| Amount (parts by mass) | SSBR1 | 100 | |
| | SSBR1-4 | | 100 |
| | Stearic acid | 2 | 2 |
| | Oil | 5 | 5 |
| | Sulfur | 1 | 1 |
| | Vulcanization accelerator NS | 1.6 | 1.6 |
| Physical properties/Evaluation | E* when wet with water (MPa) | 1.60 | 25.70 |
| | E* when dry (MPa) | 1.48 | 29.01 |
| | E* when wet with water/E* when dry | 1.08 | 0.89 |
| | Reduction of E* when wet with water | 100 | 122 |

[Table 3]

| | | Comparative Example | Example | |
|---|---|---|---|---|
| | | 2-1 | 2-1 | 2-2 |
| Amount (parts by mass) | SSBR1 | 100 | 30 | 30 |
| | SSBR1-4 | | 70 | |
| | SSBR1-5 | | | 70 |
| | Potassium acetate | 7.24 | 7.24 | 7.24 |
| | Silica | 25 | 25 | 25 |
| | Silane coupling agent | 2 | 2 | 2 |
| | Stearic acid | 2 | 2 | 2 |
| | Sulfur | 1 | 1 | 1 |
| | Vulcanization accelerator NS | 1.6 | 1.6 | 1.6 |
| | Vulcanization accelerator DPG | 1.5 | 1.5 | 1.5 |
| Physical properties/Evaluation | E* when wet with water (MPa) | 28.34 | 147.12 | 60.58 |
| | E* when dry (MPa) | 30.59 | 175.67 | 66.79 |
| | E* when wet with water/E* when dry | 0.93 | 0.84 | 0.91 |
| | Reduction of E* when wet with water | 100 | 111 | 102 |

[Table 4]

| | | Comparative Example | Example | |
|---|---|---|---|---|
| | | 3-1 | 3-1 | 3-2 |
| Amount (parts by mass) | SSBR1 | 30 | 30 | 30 |
| | SSBR2 | 70 | | |
| | SSBR2-1 | | 70 | |
| | SSBR2-2 | | | 70 |
| | Potassium acetate | 7.24 | 7.24 | 7.24 |
| | Silica | 25 | 25 | 25 |
| | Silane coupling agent | 2 | 2 | 2 |
| | Stearic acid | 2 | 2 | 2 |
| | Sulfur | 1 | 1 | 1 |
| | Vulcanization accelerator NS | 1.6 | 1.6 | 1.6 |
| | Vulcanization accelerator DPG | 1.5 | 1.5 | 1.5 |
| Physical properties/Evaluation | E* when wet with water (MPa) | 30.85 | 45.27 | 46.89 |
| | E* when dry (MPa) | 33.86 | 52.69 | 58.18 |
| | E* when wet with water/E* when dry | 0.91 | 0.86 | 0.81 |
| | Reduction of E* when wet with water | 100 | 106 | 113 |

[Table 5]

| | | Comparative Example | Example | |
|---|---|---|---|---|
| | | 4-1 | 4-1 | 4-2 |
| Amount (parts by mass) | SSBR1 | 30 | 30 | 30 |
| | BR2 | 70 | | |
| | BR2-1 | | 70 | |
| | BR2-2 | | | 70 |
| | Potassium acetate | 7.24 | 7.24 | 7.24 |
| | Silica | 25 | 25 | 25 |
| | Silane coupling agent | 2 | 2 | 2 |
| | Stearic acid | 2 | 2 | 2 |
| | Sulfur | 1 | 1 | 1 |
| | Vulcanization accelerator NS | 1.6 | 1.6 | 1.6 |
| | Vulcanization accelerator DPG | 1.5 | 1.5 | 1.5 |
| Physical properties/Evaluation | E* when wet with water (MPa) | 27.69 | 41.19 | 34.82 |
| | E* when dry (MPa) | 31.29 | 53.23 | 40.72 |
| | E* when wet with water/E* when dry | 0.88 | 0.77 | 0.86 |
| | Reduction of E* when wet with water | 100 | 114 | 103 |

Tables 2 to 5 show that the E* when wet with water is reduced relative to the E* when dry in the case of the rubber compositions containing ionic functional group-containing SBRs (SSBR1-4, SSBR1-5, SSBR2-1, SSBR2-2, BR2-1, BR2-2), compared to rubber compositions containing the corresponding polymers (SSBRl, SSBR2, BR2). As described above, each polymer showed a tendency to be softened by water when it is modified into an ionic functional group-containing polymer.

The IR spectra of Example 1-1 in which SSBR1-4 (ionic functional group-containing SBR) was used and Comparative Example 1-1 in which the corresponding SSBR1 was used, and their difference spectrum (Fig. 8) confirmed the formation of a COOH-Zn bond.

As described above, when rubber compositions for tires containing various ionic functional group-containing polymers having ion binding points (sites where ionic bonds are formed) come into contact with a wet road surface, the ion binding points are dissociated to loosen the crosslinkage, lowering the elastic modulus. Therefore, the contact area with the road surface during running on a wet road surface increases to increase the friction, which improves the loss. This is believed to improve the wet grip performance.

The present invention (1) relates to a nitrile oxide compound containing: a nitrile oxide group; and an ionic functional group.

The present invention (2) relates to the nitrile oxide compound according to the present invention (1), wherein the ionic functional group is at least one selected from the group consisting of amino groups and carboxylic acid groups.

The present invention (3) relates to the nitrile oxide compound according to the present invention (1) or (2), which is a compound containing a cyclic structure.

The present invention (4) relates to the nitrile oxide compound according to the present invention (1) or (2), which is a compound containing an aromatic ring.

The present invention (5) relates to the nitrile oxide compound according to the present invention (1) or (2), which is a compound containing a naphthalene ring.

The present invention (6) relates to an ionic functional group-containing polymer obtained by reacting the nitrile oxide compound containing a nitrile oxide group and an ionic functional group according to any one of the present inventions (1) to (5) with a polymer.

The present invention (7) relates to the ionic functional group-containing polymer according to the present invention (6), wherein the polymer is at least one selected from the group consisting of styrene-butadiene rubbers, polybutadiene rubbers, and isoprene-based rubbers.

The present invention (8) relates to a rubber composition containing the ionic functional group-containing polymer according to the present invention (6) or (7).

The present invention (9) relates to the ionic functional group-containing polymer according to the present invention (6), wherein the ionic functional group-containing polymer contains at least one selected from the group consisting of ionic functional group-containing styrene-butadiene rubbers and ionic functional group-containing styrene-butadiene rubbers.

The present invention (10) relates to the rubber composition according to the present invention (8) or (9), wherein the rubber composition contains at least one selected from the group consisting of silica and carbon black.

The present invention (11) relates to a rubber composition according to any one of the present inventions (8) to (10), which satisfies the following relationship (1 :

$$(1)\ E^*\ \text{when wet with water}/E^*\ \text{when dry} \leq 0.95$$

where E* represents a complex modulus of elasticity (MPa) 30 minutes after start of measurement at a temperature of 30°C, an initial strain of 10%, a dynamic strain of 1%, and a frequency of 10 Hz in an elongation mode for a measurement time of 30 minutes.

The present invention (12) relates to a tire produced using the rubber composition according to any one of the present inventions (8) to (11).

## Claims

1. A nitrile oxide compound comprising:

   a nitrile oxide group; and
   an ionic functional group.

2. The nitrile oxide compound according to claim 1,
   wherein the ionic functional group is at least one selected from the group consisting of amino groups and carboxylic acid groups.

3. The nitrile oxide compound according to claim 1 or 2, which is a compound containing a cyclic structure.

4. The nitrile oxide compound according to claim 1 or 2, which is a compound containing an aromatic ring.

5. The nitrile oxide compound according to claim 1 or 2, which is a compound containing a naphthalene ring.

6. An ionic functional group-containing polymer obtained by reacting the nitrile oxide compound comprising a nitrile oxide group and an ionic functional group according to any one of claims 1 to 5 with a polymer.

7. The ionic functional group-containing polymer according to claim 6,
   wherein the polymer is at least one selected from the group consisting of styrene-butadiene rubbers, polybutadiene rubbers, and isoprene-based rubbers.

8. A rubber composition comprising
   the ionic functional group-containing polymer according to claim 6 or 7.

9. The ionic functional group-containing polymer according to claim 6,
   wherein the ionic functional group-containing polymer contains at least one selected from the group consisting of ionic functional group-containing styrene-butadiene rubbers and ionic functional group-containing styrene-butadiene rubbers.

10. The rubber composition according to claim 8 or 9,
    wherein the rubber composition contains at least one selected from the group consisting of silica and carbon black.

11. The rubber composition according to any one of claims 8 to 10, which satisfies the following relationship (1):

$$(1)\ E^*\ \text{when wet with water}/\ E^*\ \text{when dry} \leq 0.95$$

where E* represents a complex modulus of elasticity (MPa) 30 minutes after start of measurement at a temperature of 30°C, an initial strain of 10%, a dynamic strain of 10, and a frequency of 10 Hz in an elongation mode for a measurement time of 30 minutes.

**12.** A tire produced using the rubber composition according to any one of claims 8 to 11.

**Amended claims under Art. 19.1 PCT**

**1.** (Amended). A nitrile oxide compound comprising:

a nitrile oxide group; and
an ionic functional group,
the nitrile oxide compound containing a naphthalene ring,
the ionic functional group being at least one selected from the group consisting of amino groups and carboxylic acid groups.

**2.** (Cancelled)

**3.** (Cancelled)

**4.** (Cancelled)

**5.** (Cancelled)

**6.** (Amended). An ionic functional group-containing polymer obtained by reacting the nitrile oxide compound comprising a nitrile oxide group and an ionic functional group according to claim 1 with a polymer.

**7.** The ionic functional group-containing polymer according to claim 6,
wherein the polymer is at least one selected from the group consisting of styrene-butadiene rubbers, polybutadiene rubbers, and isoprene-based rubbers.

**8.** A rubber composition comprising
the ionic functional group-containing polymer according to claim 6 or 7.

**9.** (Amended). The rubber composition according to claim 8,
the ionic functional group-containing polymer contains at least one selected from the group consisting of ionic functional group-containing styrene-butadiene rubbers and ionic functional group-containing polybutadiene rubbers.

**10.** The rubber composition according to claim 8 or 9,
wherein the rubber composition contains at least one selected from the group consisting of silica and carbon black.

**11.** The rubber composition according to any one of claims 8 to 10, which satisfies the following relationship (1):

$$\text{(1) E* when wet with water/E* when dry} \leq 0.95$$

where E* represents a complex modulus of elasticity (MPa) 30 minutes after start of measurement at a temperature of 30°C, an initial strain of 10%, a dynamic strain of 1%, and a frequency of 10 Hz in an elongation mode for a measurement time of 30 minutes.

**12.** A tire produced using the rubber composition according to any one of claims 8 to 11.

**Statement under Art. 19.1 PCT**

Claim 1: This claim has been amended on the basis of original claims 2 to 5.
Claims 2 to 5: These claims are cancelled.

Claim 6: This claim has been amended to change its dependency as a result of the cancellation of claims 2 to 5.

Claim 9: This claim has been amended to include the limitation based on paragraph [0050] of the specification as filed and change its dependency to change the preamble.

FIG.1

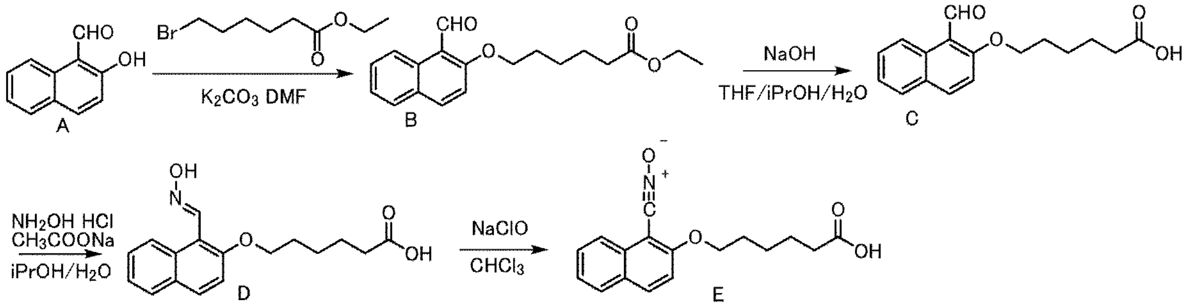
A
B
C
D
E
$K_2CO_3$ DMF
NaOH
THF/iPrOH/$H_2O$
$NH_2OH$ HCl
$CH_3COONa$
iPrOH/$H_2O$
NaClO
$CHCl_3$

FIG.2

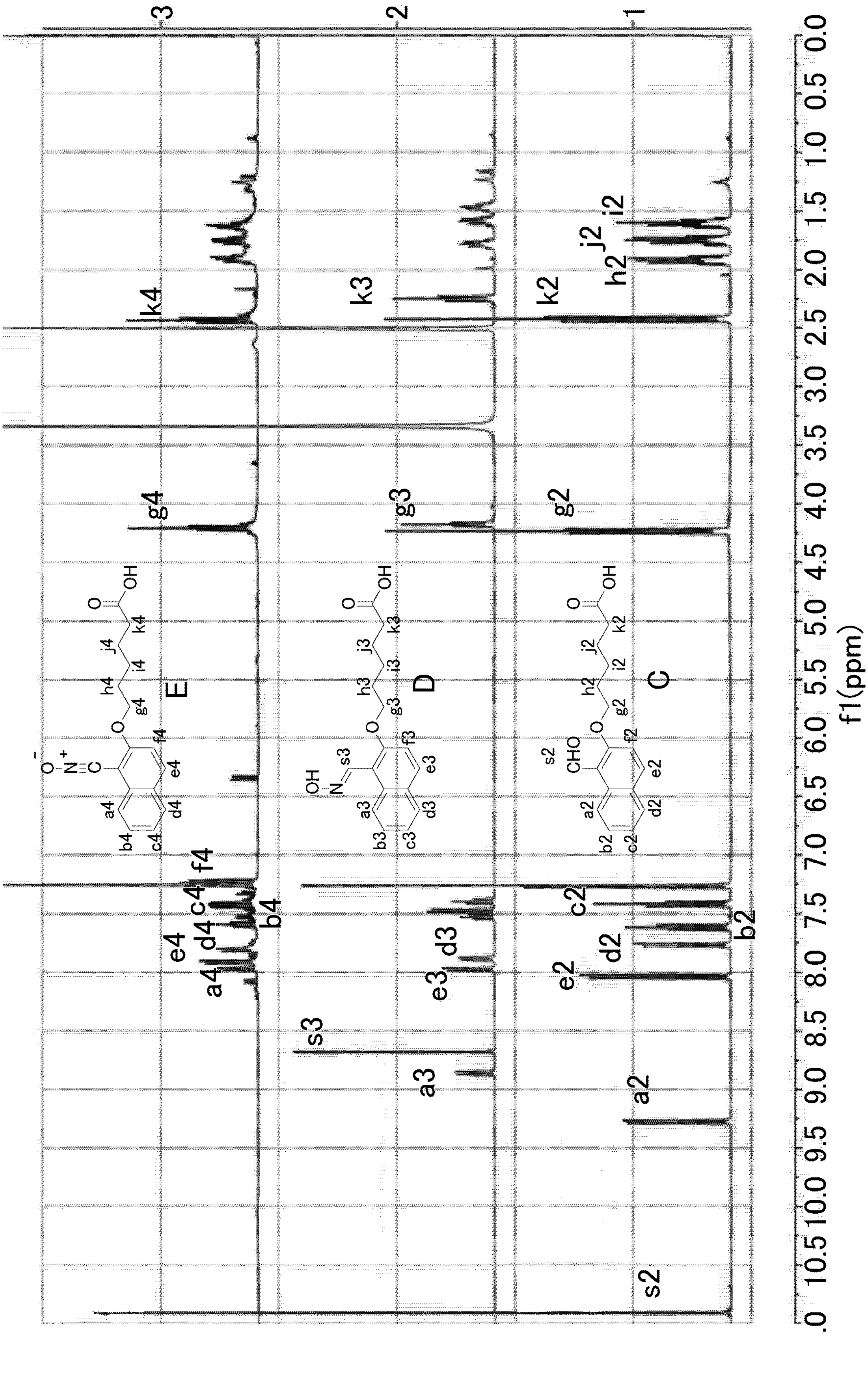
f1(ppm)
3
2
1
0.0 0.5 1.0 1.5 2.0 2.5 3.0 3.5 4.0 4.5 5.0 5.5 6.0 6.5 7.0 7.5 8.0 8.5 9.0 9.5 10.0 10.5 11.0
E
D
C
k4
g4
a4
e4
d4
c4
f4
b4
k3
g3
e3
d3
a3
s3
k2
h2
j2
i2
g2
e2
d2
c2
b2
a2
s2

FIG.3

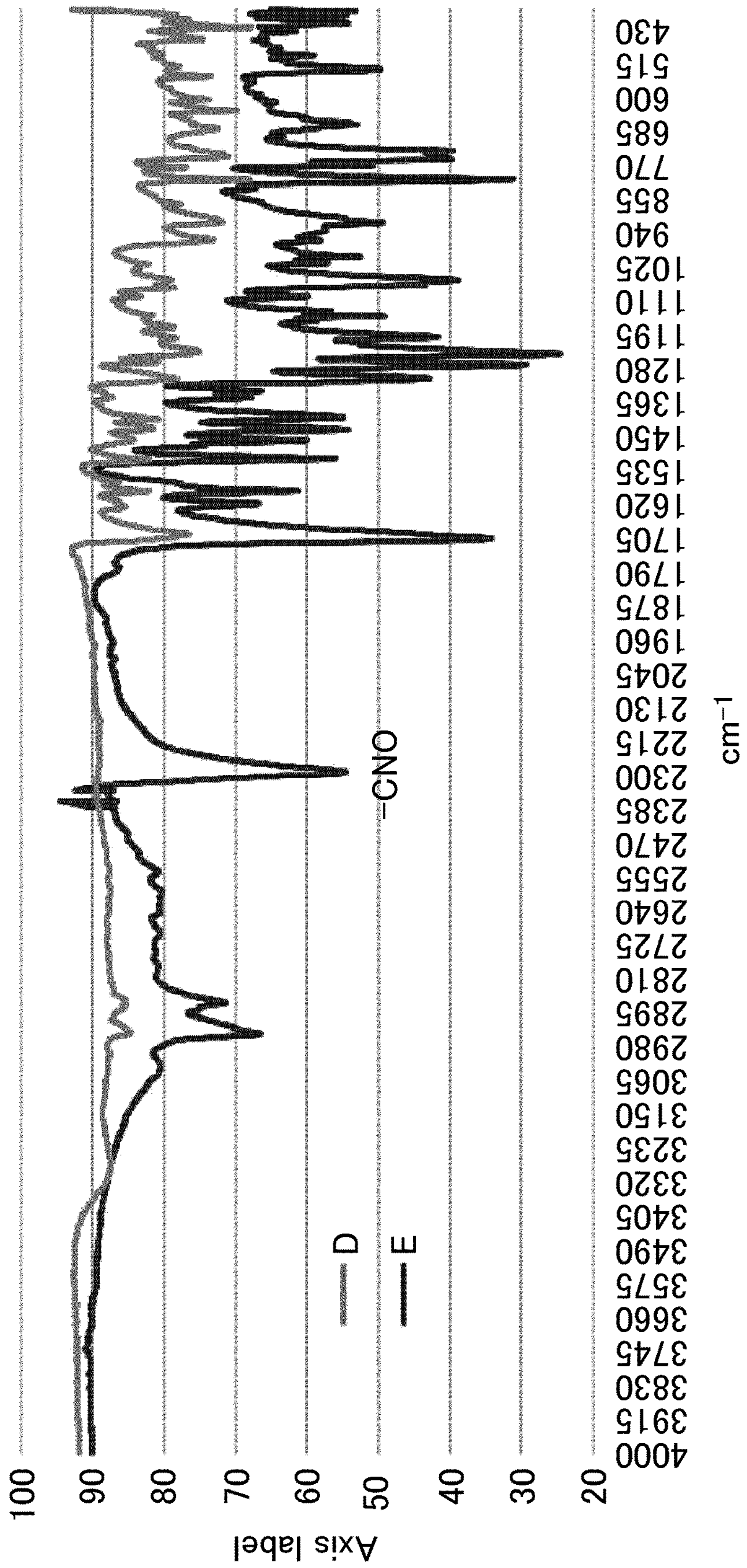

FIG.4

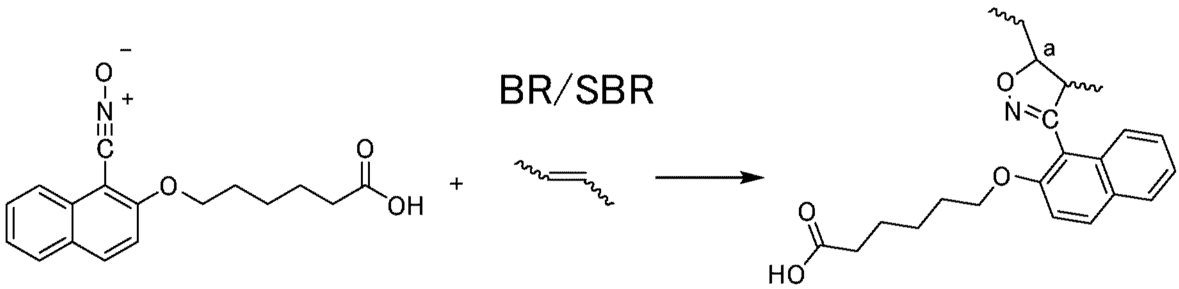

FIG.5

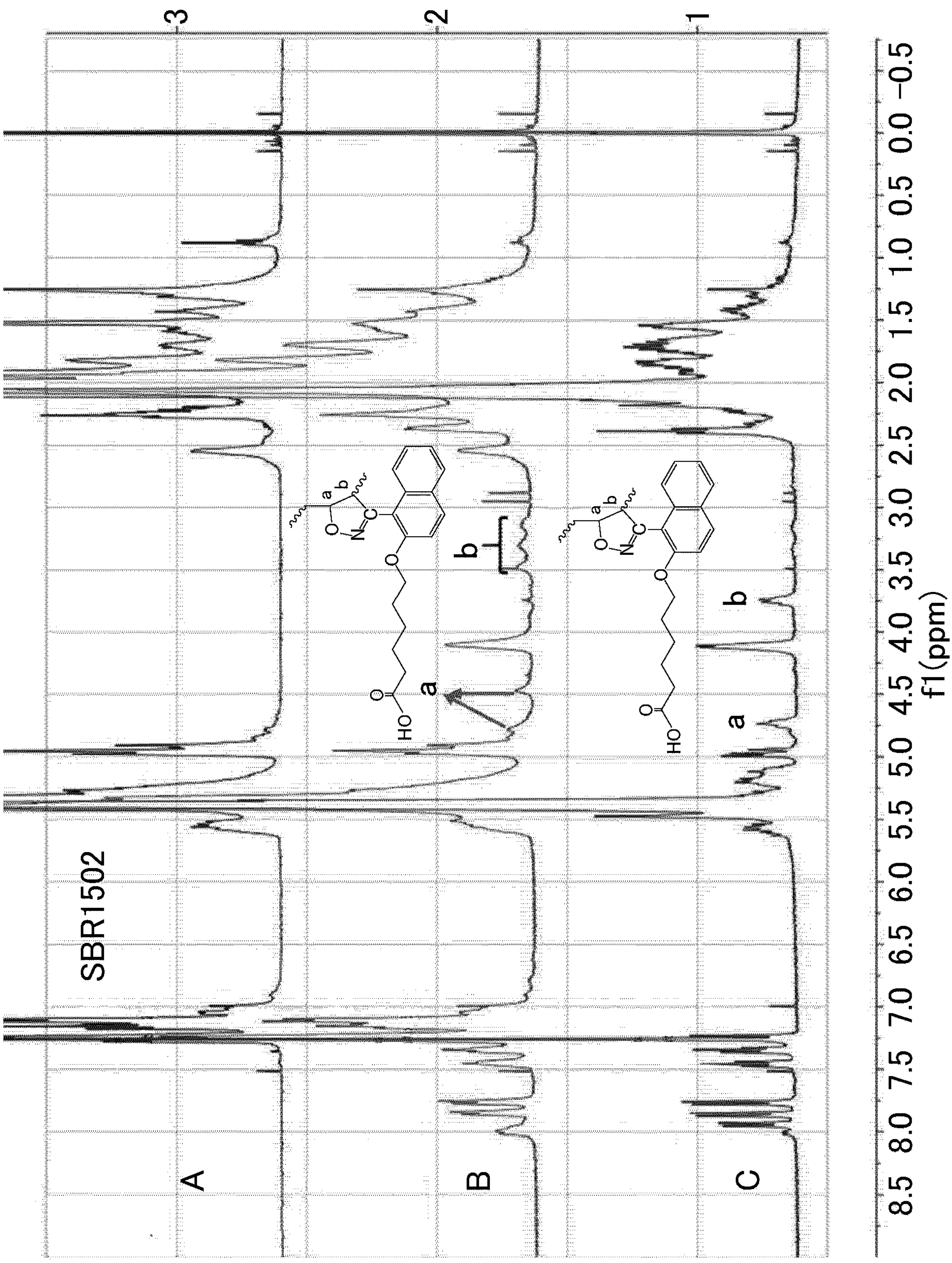
SBR1502
A
B
C
f1(ppm)
8.5 8.0 7.5 7.0 6.5 6.0 5.5 5.0 4.5 4.0 3.5 3.0 2.5 2.0 1.5 1.0 0.5 0.0 -0.5
3
2
1

FIG.6

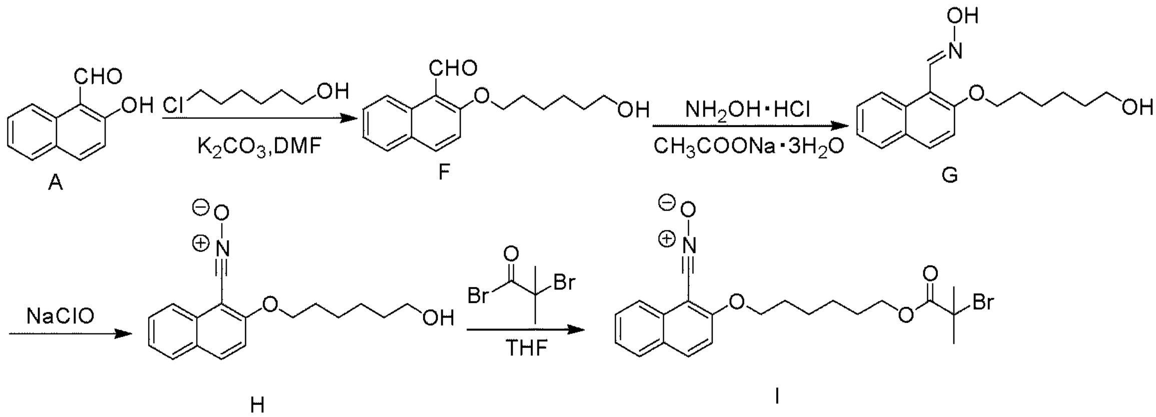


A F G H I

FIG.7

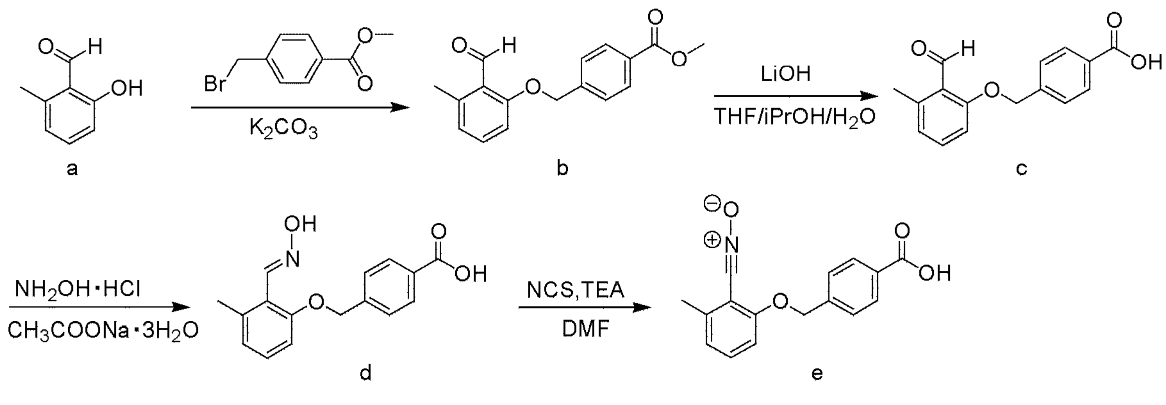


a b c d e

FIG.8

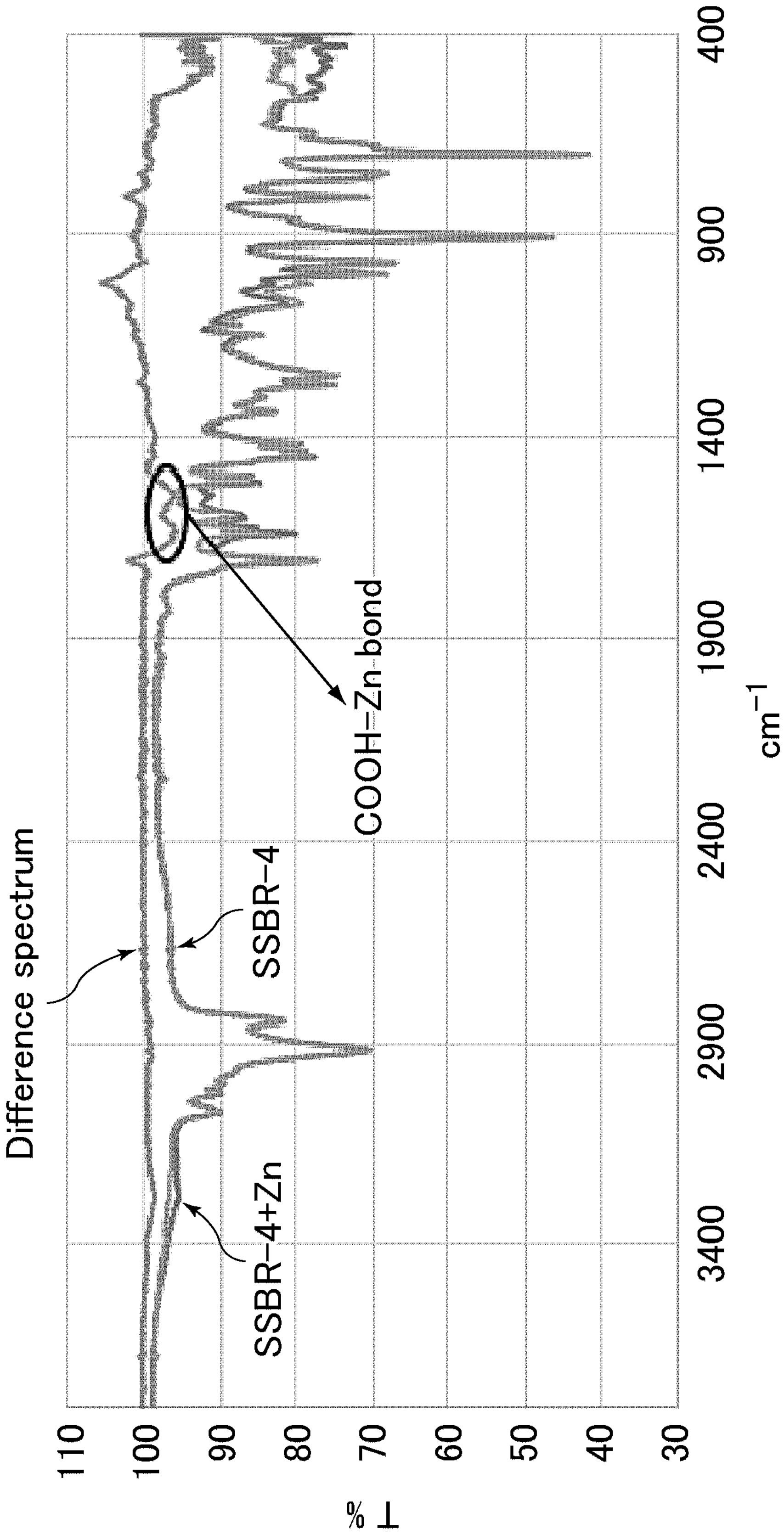
Difference spectrum
SSBR-4
SSBR-4+Zn
COOH-Zn bond
T %
cm$^{-1}$
110
100
90
80
70
60
50
40
30
3400
2900
2400
1900
1400
900
400

## INTERNATIONAL SEARCH REPORT

International application No. **PCT/JP2022/003943**

**A. CLASSIFICATION OF SUBJECT MATTER**

***B60C 1/00***(2006.01)i; ***C07C 291/06***(2006.01)i; ***C08C 19/22***(2006.01)i; ***C08F 36/04***(2006.01)i; ***C08L 15/00***(2006.01)i
FI: C07C291/06 CSP; C08F36/04; C08L15/00; B60C1/00 Z; C08C19/22

According to International Patent Classification (IPC) or to both national classification and IPC

**B. FIELDS SEARCHED**

Minimum documentation searched (classification system followed by classification symbols)

B60C1/00; C07C291/06; C08C19/22; C08F36/04; C08L15/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2017-160164 A (DAIKIN INDUSTRIES, LTD.) 14 September 2017 (2017-09-14) paragraphs [0001], [0030], [0031], [0094]-[0096], [0101]-[0106], [0108]-[0123], [0125]-[0129] | 1-12 |
| Y | | 1-12 |
| X | JP 2017-501240 A (COMPAGNIE GENERALE DES ETABLISSEMENTS MICHELIN) 12 January 2017 (2017-01-12) paragraphs [0001], [0012]-[0015], [0019], [0050], [0054], [0055], [0072]-[0075], [0093]-[0101], [0107]-[0111] | 1-12 |
| X | JP 2011-057946 A (BRIDGESTONE CORP.) 24 March 2011 (2011-03-24) paragraphs [0001], [0052]-[0061], [0065], [0066], [0075]-[0082] | 1-12 |
| X | WO 1997/003034 A2 (THE DOW CHEMICAL CO.) 30 January 1997 (1997-01-30) p. 3, line 1 to p. 4, line 7, p. 6, lines 1-10, p. 7, line 10 to p. 8, line 10, p. 9, lines 13-15, example 4 | 1-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 March 2022** | **29 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
PCT/JP2022/003943

C. DOCUMENTS CONSIDERED TO BE RELEVANT

| Category* | Citation of document, with indication, where appropriate, of the relevant passages | Relevant to claim No. |
|---|---|---|
| X | WO 2019/107450 A1 (MITSUBISHI CHEMICAL CORP.) 06 June 2019 (2019-06-06) paragraphs [0001], [0052], [0066], [0068], [0072], [0109], [0111], [0159], [0160], [0206]-[0212] | 1-4, 6-9 |
| X | WO 2016/143870 A1 (DAIKIN INDUSTRIES, LTD.) 15 September 2016 (2016-09-15) paragraphs [0001], [0036], [0042], [0043], [0046], [0051], [0059], [0128], [0131], [0137], [0393], [0398], [0404]-[0407], [0413], [0414] | 1-9, 11 |
| X | US 5710290 A (LYSENKO, Zenon) 20 January 1998 (1998-01-20) column 7, line 3 to column 8, line 8, example 1 | 1-3, 6-9 |
| X | JP 2004-527475 A (CHUGAI SEIYAKU KABUSHIKI KAISHA) 09 September 2004 (2004-09-09) paragraphs [0085], [0088] | 1, 2 |
| Y | JP 2018-024768 A (BRIDGESTONE CORP.) 15 February 2018 (2018-02-15) paragraphs [0001], [0011], [0039] | 1-12 |
| Y | JP 2014-201629 A (THE YOKOHAMA RUBBER CO., LTD.) 27 October 2014 (2014-10-27) paragraphs [0001], [0006], [0010], [0011], [0022] | 1-12 |
| Y | WO 2001/083566 A1 (SENTAN KAGAKU GIJUTSU INCUBATION CENTER KK) 08 November 2001 (2001-11-08) p. 1, lines 4-7, p. 12, lines 24, 25, p. 13, lines 17, 18 | 1-12 |

Form PCT/ISA/210 (second sheet) (January 2015)

**INTERNATIONAL SEARCH REPORT**
**Information on patent family members**

International application No.
**PCT/JP2022/003943**

| Patent document cited in search report | Publication date (day/month/year) | Patent family member(s) | Publication date (day/month/year) |
|---|---|---|---|
| JP 2017-160164 A | 14 September 2017 | (Family: none) | |
| JP 2017-501240 A | 12 January 2017 | US 2016/0263943 A1<br>paragraphs [0001], [0035]-[0044], [0055], [0107], [0108], [0114], [0124]-[0126], [0141]-[0150], [0154]-[0156]<br>EP 3060413 A1<br>WO 2015/059274 A1 | |
| JP 2011-057946 A | 24 March 2011 | (Family: none) | |
| WO 1997/003034 A2 | 30 January 1997 | EP 0842147 A2 | |
| WO 2019/107450 A1 | 06 June 2019 | US 2020/0283429 A1<br>paragraphs [0001], [0169], [0200], [0201], [0205], [0216], [0281], [0285], [0385], [0492]-[0497]<br>EP 3719003 A1 | |
| WO 2016/143870 A1 | 15 September 2016 | US 2018/0050983 A1<br>paragraphs [0001], [0107], [0113], [0114], [0117], [0122], [0178]-[0183], [0341], [0344], [0350], [0630], [0640], [0661]-[0670], [0681]-[0684]<br>EP 3269708 A1 | |
| US 5710290 A | 20 January 1998 | (Family: none) | |
| JP 2004-527475 A | 09 September 2004 | US 2004/0082576 A1<br>paragraphs [0125], [0128]<br>EP 1353662 A2<br>WO 2002/058698 A2 | |
| JP 2018-024768 A | 15 February 2018 | (Family: none) | |
| JP 2014-201629 A | 27 October 2014 | (Family: none) | |
| WO 2001/083566 A1 | 08 November 2001 | US 2003/0138398 A1<br>paragraphs [0001], [0081], [0089]<br>EP 1283218 A1 | |

Form PCT/ISA/210 (patent family annex) (January 2015)